# EUROPEAN PATENT SPECIFICATION

(11) **EP 4 103 733 B1**
(45) Date of publication and mention of the grant of the patent: **22.04.2026**
(21) Application number: 20918941.4
(22) Date of filing: 08.09.2020
(51) Int. Cl.: C07D 455/03, A61K 31/4745, A61K 45/06, A61P 25/34

(54) **BERBERINE COMPOUNDS, BERBERINE COMPOSITIONS, AND METHODS FOR ADMINISTRATION THEREOF**
BERBERINDERIVATE, BERBERINZUSAMMENSETZUNGEN UND VERFAHREN ZU DEREN VERABREICHUNG
COMPOSÉS DE BERBÉRINE, COMPOSITIONS DE BERBÉRINE ET LEURS PROCÉDÉS D'ADMINISTRATION

(30) Priority: 11.02.2020 US 202062972677 P
(43) Date of publication of application: 21.12.2022
(73) Proprietor: Dignity Health, San Francisco, CA 94107 (US)
(72) Inventor: WU, Jie, San Francisco, California 94107 (US); OLSEN, Mark, San Francisco, California 94107 (US)
(74) Representative: Hutter, Anton
(86) International application number: PCT/US2020/049671
(87) International publication number: WO 2021/162750

(56) References cited:
- WO-A1-2011/139983
- CN-A- 107 875 151
- US-A1- 2008 124 404
- US-A1- 2015 164 870
- US-A1- 2019 185 900
- WU C ET AL: "Tetrahydroberberine blocks ATP-sensitive potassium channels in dopamine neurons acutely-dissociated from rat substantia nigra pars compacta", NEUROPHARMACOLOGY, ELSEVIER, AMSTERDAM, NL, vol. 59, no. 7-8, December 2010 (2010-12-01), pages 567 - 572, XP027430916, ISSN: 0028-3908, DOI: 10.1016/J.NEUROPHARM.2010.08.018
- DATABASE PUBCHEM SUBSTANCES 16 September 2004 (2004-09-16), "(S)-Canadine", XP055849259, retrieved from NCBI Database accession no. CID21171

## Description

### FIELD OF THE DISCLOSURE

The present disclosure generally relates to compounds and berberine compositions containing such compounds that are useful for reducing nicotine dependency. Additionally, the present disclosure relates to the compounds for use in methods for administering the berberine compositions and, particularly, to methods for treating nicotine addiction.

### BACKGROUND OF THE DISCLOSURE

Tobacco smoking is one of the leading preventable cause of disease, disability, and death in the U.S. Typically, tobacco smoking relating deaths account for more than 400,000 deaths each year in the U.S. Nicotine is the principal alkaloid in tobacco and is primarily responsible for tobacco dependence. The initiation and maintenance of tobacco dependence in a human is due to certain bio-behavioral and neuromolecular mechanisms. Nicotinic acetylcholine receptors (nAChRs) in humans are the initial binding sites for nicotine. The binding of nicotine to nAChRs modulates the brain's "reward" function by triggering dopamine release in the human brain.

Although a variety of psychopharmacological effects contribute to the reinforcing action, the existence of a mesolimbic dopaminergic pathway for nicotinic reward is the predominant hypothesis. The mesolimbic dopaminergic pathway originates in the ventral tegmental area (VTA) of the midbrain and projects to forebrain structures including the prefrontal cortex and to limbic areas, such as the olfactory tubercle, the amygdala, the septal region, and the nucleus accumbens. Many studies have indicated that dopamine release in the nucleus accumbens of the human brain is "rewarding" or signals an encounter with a "reward" from the environment.

Currently, the U.S. Food and Drug Administration ("FDA") has approved several medications for addressing nicotine addiction and facilitating tobacco smoking cessation. Two common groups of FDA approved medications for addressing nicotine addiction include nicotine replacement medications ("NRT") and α4β2 nAChR partial agonist varenicline. Varenicline containing products often have adverse side effects, such as nausea, insomnia, abnormal dreams, and headaches. Depression, suicidal thoughts, and suicidal action have also been associated with varenicline containing products. Individuals trying to quit smoking tobacco can be helped with pharmacotherapy, sometimes combined with behavioral interventions, through formal settings, such as smoking cessation clinics. However, about 75 to 85% of people that use these intervention programs relapse within 6 months. Therefore, more efficacious medications for nicotine addiction is needed.

US 2008/124404 A1, WO 2011/139983 A1 and WU C et al. ("Tetrahydroberberine blocks ATP-sensitive potassium channels in dopamine neurons acutely-dissociated from rat substantia nigra pars compacta", Neuropharmacology, 2010 Dec; 59(7-8): 567-72) disclose the compound tetrahydroberberine (THB). US 2015/164870 A1 and CN107875151A disclose berberine derivatives.

### SUMMARY OF THE DISCLOSURE

The invention is as defined in the independent claims. Preferred embodiments of the invention are recited in the dependent claims. Various other embodiments are included in the description, not all of which are encompassed by the claims, but are included to aid the reader's understanding of the claimed invention.

### FIGURES OF THE DISCLOSURE

Implementation of the present technology will now be described, by way of example only, with reference to the attached figures, wherein:
FIGS. 1A-1C are graphs illustrating the effect of THPBs on human α4β2-nAChR-mediated currents in heterologously expressed SH-EP1 cell line according to aspects of the disclosure.
FIG. 2A is a graph of the affinity for THB and *I*-SPD for H-EBDN binding according to aspects of the disclosure.
FIG. 2B is graph showing ⁸⁶Rb⁺ Efflux mM Carb response to THB and *I*-SPD in accordance with aspects of the disclosure.
FIGS. 3A-3C provide graphs showing that 30 µM THB inhibited α4β2-nAChR function in both rat and human cells according to aspects of the disclosure.
FIG. 4 is a graph showing THB inhibited human α4β2 nAChR-mediated currents in a concentration-dependent manner in accordance with aspects of the disclosure.
FIG. 5A is a graph of traces of mEPSCs before, during NIC exposure, and during THB plus NIC co-exposure according to aspects of the disclosure.
FIG. 5B is a histograph showing the nicotine-induced increase in mEPSC frequency in accordance with aspects of the disclosure.
FIG. 5C is a bar graph comparing mEPSC frequency (normalized) among control, nicotine and nicotine plus THB exposures according aspects of the disclosure.
FIG. 5D is bar graph showing that nicotine and nicotine plus THB did not affect mEPSC amplitude in accordance with aspects of the disclosure.
FIG. 6A is a trace showing THB enhanced VTA DA neuron firing rate and bursting fraction according to aspects of the disclosure.
FIG. 6B is a graph showing the firing pattern changes after injection of THB in accordance with aspects of the disclosure.
FIGS. 6C and 6D are graphs of power spectral analysis after an injection of THB according to aspects of the disclosure.
FIG. 6D is a bar graph showing the effects of THB on VTA DA neuronal firing and the effects of THB on nicotine-induced increase of VTA DA neuron firing in accordance with aspects of the disclosure.

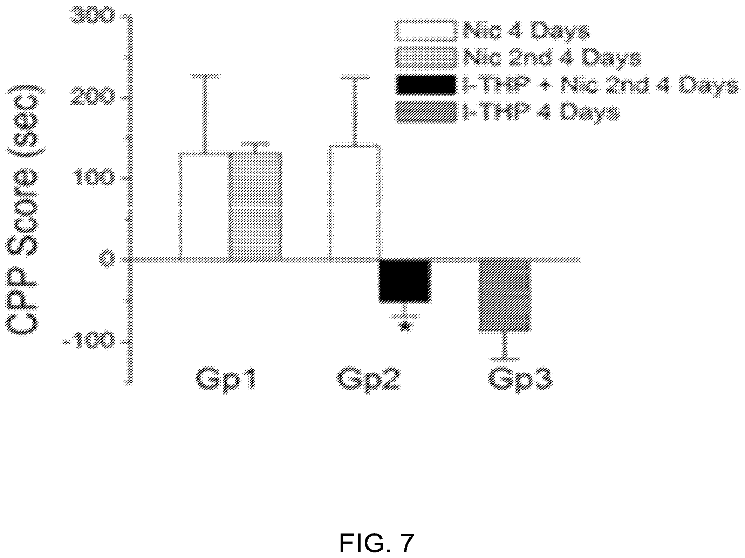

FIG. 7 is a bar graph showing that I-THP counteracts nicotine-induced CPP according to aspects of the disclosure.
FIG. 8A shows typical traces illustrating the changes of 3 µM nicotine (NIC, about the EC50 concentration)-induced currents with co-application of THB6 with different concentrations in accordance with aspects of the disclosure.
FIGS. 8B-8D provide graphs of concentration-inhibition efficacy curves by measuring the peak current, the steady-state current, and the net charge (current response area/Cm) according to aspects of the disclosure.
FIG. 9A provides a graph of traces of co-application of THB6 on ACh-induced current in accordance with aspects of the disclosure.
FIG. 9B provides a graph of traces of the effect of 1 min pretreatment of THB6 on ACh-induced current according to aspects of the disclosure.
FIG. 9C provides a bar graph showing the statistic result of THB6 inhibiting the peak compound of ACh-induced currents in accordance with aspects of the disclosure.
FIG. 10A is a graph of traces of ACh-induced whole-cell currents in human α4β2-nAChRs transfected in human SH-EP1 cells according to aspects of the disclosure.
FIG. 10B is a graph of traces of ACh-induced whole-cell currents in mouse α4β2-nAChRs transfected in VTA dopamine neurons in accordance with aspects of the disclosure.
FIGS. 10C and 10D provide bar graph showing the statistic result that THB6 inhibits both peak and steady-state compounds of ACh-induced currents according to aspects of the disclosure.
FIG. 11A is a graph of traces of extracellular action potential firing using cell-attached patch recording mode in mouse VTA neurons in accordance with aspects of the disclosure.
FIG. 11B is bar graph showing the statistic result of the normalized firing rate of with THB6 compounds according to aspects of the disclosure.
FIG. 12A provides an image of the VTA slice in accordance with an aspect of the disclosure.
FIG. 12B is a depiction of the location of the recording electrode track according to an aspect of the disclosure.
FIG. 12C is a graph depicting a single extracellular action potential in accordance with aspects of the disclosure.
FIGS. 12D and 12E are graphs showing that THB6 prevented nicotine-induced increase of firing rate according to aspects of the disclosure.
FIGS. 12F-12H are graphs depicting the neuronal firing rate in response to THB6 according to aspects of the disclosure.
FIGS. 12I and 12J are histogram and bar graphs showing neuronal firing rate at 2 minutes and after 8 minutes of injections containing nicotine and/or THB6 in accordance with aspects of the disclosure.
FIG. 13A is a graph showing that an injection of THB6 alone did not alter locomotor activity in mice according to aspects of the disclosure.
FIG. 13B shows the statistic result that THB6 prevents nicotine-induced increase of mouse locomotor activity in accordance with aspects of the disclosure.

It should be understood that the various aspects are not limited to the arrangements and instrumentality shown in the drawings.

### DETAILED DESCRIPTION OF THE DISCLOSURE

Aspects of the disclosure generally compounds and berberine compositions containing such compounds that are useful for reducing nicotine dependency. The compounds and berberine compositions thereof may reduce nicotine urges and, thus, reduce nicotine dependency. For instance, the compounds and berberine compositions thereof may possess an equipotent effect on D1-type and D2-type DA receptor binding. The compounds according to the instant disclosure may, thus, be effective in blocking nicotine-induced DA release, which is the major cellular mechanism of nicotine reward and dependence.

In some aspects, the berberine compositions include certain combinations of compounds in specific amounts to simultaneously reduce nicotine dependency while reducing adverse side effects associated with tetrahydroberberine ("THB"). Preferably, the berberine compositions provide an enhanced reduction in nicotine dependency as compared to compositions containing solely THB, based on equal amounts of the active ingredients.

In accordance with one aspect of the disclosure, provided are compounds having a structure according to Formula (I): 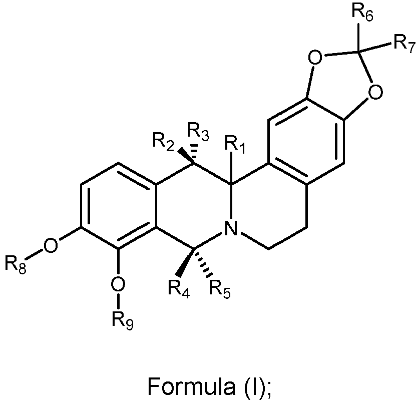 wherein
- R₁, R₂, R₃, R₄, and Rs are independently H or D;
- R₆ and R₇ are independently hydrogen, deuterium, halogen, C₄ to C₈ unsubstituted aryl, C₄ to C₈ substituted aryl, C₂ to C₆ unsubstituted heterocycle, C₂ to C₆ substituted heterocycle, C₁ to C₆ unsubstituted alkyl, C₁ to C₆ substituted alkyl, C₃-C₁₀ unsubstituted cycloalkyl, C₃-C₁₀ substituted cycloalkyl, or R₆ and R₇ are taken together as =O or =S; and
- R₈ and R₉ are independently selected from a group consisting of methyl, CHF₂, and CF₃.

The compounds of Formula (I) may have a structure where at least one of R₂ and R₃ is deuterium and at least one of R₄ and R₅ is deuterium. In some cases, R₁, at least one of R₂ and R₃, and at least one of R₄ and R₅ are deuteriums. Although the compounds of Formula (I) typically include at least one of R₁, R₂, R₃, R₄, and R₅ that is hydrogen, in some instances each of R₁, R₂, R₃, R₄, and R₅ are deuteriums. In at least one instance, the compounds of Formula (I) have a structure where R₁ is deuterium.

R₆ and R₇ of the compounds of Formula (I) may independently be hydrogen, deuterium, halogen, C₄ to C₈ unsubstituted aryl, C₄ to C₈ substituted aryl, C₂ to C₆ unsubstituted heterocycle, C₂ to C₆ substituted heterocycle, C₁ to C₆ unsubstituted alkyl, C₁ to C₆ substituted alkyl, C₃ to C₁₀ unsubstituted cycloalkyl, C₃ to C₁₀ substituted cycloalkyl, or R₆ and R₇ are taken together as =O or =S. R₆ and R₇ may be chosen from C₄ to C₈ unsubstituted or substituted aryl, such as C₅ to C₈ aryl, C₆ to C₈ aryl, C₇ to C₈ aryl, C₅ to C₇ aryl, or C₅ to C₆ aryl. The compounds of Formula (I) may have a structure where at least one of R₆ and R₇ is a C₄ to C₆ unsubstituted or substituted heterocycle containing at least one nitrogen. For example, R₆ and R₇ may independently be a C₄ to C₆ unsubstituted or substituted heterocycle chosen from heterocycles having 4 carbons, 5 carbons, or 6 carbons. The heterocycles may contain at least one nitrogen silicon, oxygen, phosphorous, and/or sulfur. Additionally or alternatively, R₆ and R₇ of the compounds of Formula (I) may independently be C₁ to C₆ unsubstituted or substituted alkyls including, e.g., C₁ to C₅ alkyls, C₁ to C₄ alkyls, C₁ to C₃ alkyls, C₁ to C₂ alkyls, C₂ to C₆ alkyls, C₃ to C₆ alkyls, C₄ to C₅ alkyls, or C₅ to C₆ alkyls. In some instances, R₆ and R₇ may independently be methyl, ethyl, n-propyl, i-propyl, n-butyl, or t-butyl. R₆ and R₇ of the compounds of Formula (I) may independently be C₃ to C₁₀ unsubstituted or substituted cycloalkyl, such as C₃ to C₉ cycloalkyl, C₃ to C₈ cycloalkyl, C₃ to C₇ cycloalkyl, C₃ to C₆ cycloalkyl, C₃ to C₅ cycloalkyl, C₃ to C₄ cycloalkyl, C₄ to C₁₀ cycloalkyl, C₅ to C₁₀ cycloalkyl, C₆ to C₁₀ cycloalkyl, C₇ to C₁₀ cycloalkyl, C₈ to C₁₀ cycloalkyl, or C₉ to C₁₀ cycloalkyl. In some cases, R₆ and R₇ may be taken together as =O or =S.

R₈ and R₉ may independently be selected from methyl, CF₂, and CF₃. Preferably, at least one of R₈ and R₉ is methyl. In some cases, R₈ and R₉ are both methyls. In other cases, at least one of R₈ and R₉ is CF₂, and/or CF₃.

In at least one embodiment, the compound of Formula (I) has a structure according to Formula (II):

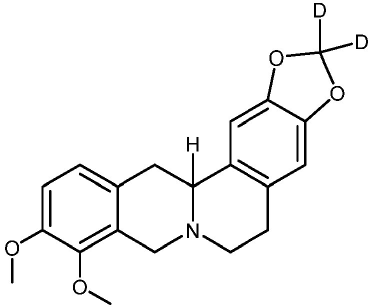

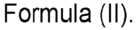

In other embodiments, the compound of Formula (I) has a structure according to Formula (III):

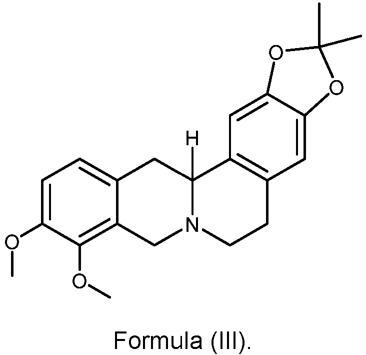

In further embodiments, the compound of Formula (I) has a structure according to Formula (IV):

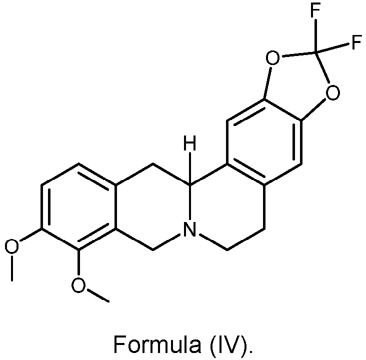

In yet further embodiments, the compound of Formula (I) has a structure according to Formula (V):

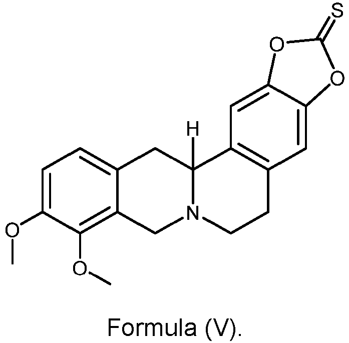

In yet other embodiments, the compound of Formula (I) has a structure according to Formula (VI):

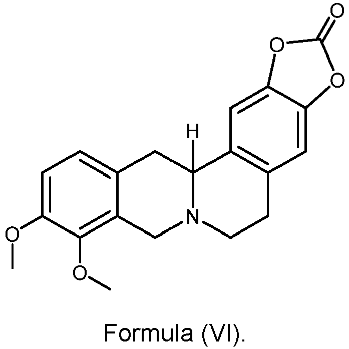

In yet additional embodiments, the compound of Formula (I) has a structure according to Formula (VII):

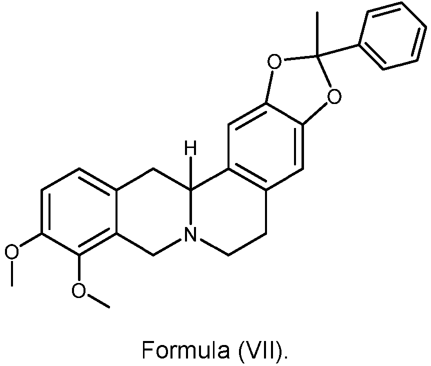

The compound of Formula (I) may be a mixture of diastereomers, a single diastereomer, a racemic mixture, or an enantiomer. In some cases the compound of Formula (I) is a (R) enantiomer, a (S) enantiomer, or a racemic mixture.

The compounds of Formula (I) may be a free base or a salt. When the compound is in a salt form, the salt may be a pharmaceutically acceptable salt. Pharmaceutically acceptable salts may include, without limitation, hydrochloride, hydrobromide, phosphate, sulfate, methanesulfonate, acetate, formate, tartaric acid, bitartrate, stearate, phthalate, hydroiodide, lactate, monohydrate, mucate, nitrate, phosphate, salicylate, phenylpropionate, isobutyrate, hypophosphite, maleic, malic, citrate, isocitrate, succinate, lactate, gluconate, glucuronate, pyruvate, oxalate, fumarate, propionate, aspartate, glutamate, benzoate, terephthalate, and the like. In other embodiments, the pharmaceutically acceptable salt includes an alkaline or alkaline earth metal ion salt. In particular, sodium, potassium or other pharmaceutically acceptable inorganic salts may be used. The salt forms may be amorphous or in various polymeric forms including hydrates, or solvates with alcohols or other solvents.

R₁, R₂, R₃, R₄, R₅, R₆, R₇, R₈, and/or R₉ of compounds of Formula (I) may, in some instances, be substituted groups or unsubstituted groups. In some cases, R₁, R₂, R₃, R₄, R₅, R₆, R₇, R₈, and/or R₉ may be substituted with at least one atom other than hydrogen, including moieties in which a carbon chain atom is substituted with a heteroatom such as nitrogen, oxygen, silicon, phosphorous, boron, or a halogen atom, and moieties in which the carbon chain comprises additional substituents. These substituents include alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro and thio groups.

In another aspect, provided are berberine compositions that comprise an amount of a compound of Formula (I) or salt thereof: 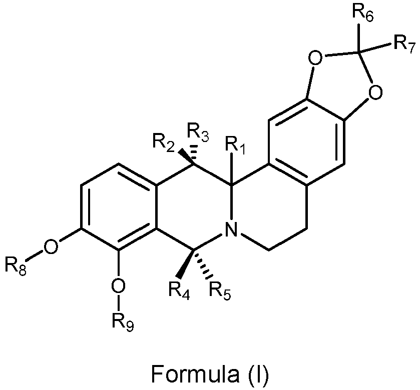 wherein
- R₁, R₂, R₃, R₄, and R₅ are independently H or D;
- R₆ and R₇ are independently hydrogen, deuterium, halogen, C₄ to C₈ unsubstituted aryl, C₄ to C₈ substituted aryl, C₂ to C₆ unsubstituted heterocycle, C₂ to C₆ substituted heterocycle, C₁ to C₆ unsubstituted alkyl, C₁ to C₆ substituted alkyl, C₃-C₁₀ unsubstituted cycloalkyl, C₃-C₁₀ substituted cycloalkyl, or R₆ and R₇ are taken together as =O or =S; and
- R₈ and R₉ are independently selected from a group consisting of methyl, CHF₂, and CF₃.

The berberine composition may include any of the compounds of Formula (I) as discussed above. Preferably, the amount of compounds of Formula (I) in the berberine composition is a therapeutically effective amount. As used herein "therapeutically effective amount" or "therapeutically effective dosage" refers to an amount that is effective to achieve a desired therapeutic result. In some embodiments, the desired therapeutic result is a reduction of nicotine dependency and/or a reduction of nicotine urges. For example, the therapeutically effective amount may be an amount that reduces the nicotine dependency and/or urges by at least 10%, preferably 20% or more, preferably 25% or more, preferably 30% or more, preferably 35% or more, preferably 40% or more, preferably 45% or more, preferably 50% or more, etc.

In some cases, the amount of compound of Formula (I) present in berberine composition is more than about 1 µg. For example, the berberine composition may comprise an amount of compounds of Formula (I) of about 2 µg or more, about 5 µg or more, about 10 µg or more, about 100 µg or more, about 500 µg or more, about 1000 µg or more, about 1500 µg or more, about 2000 µg or more, about 2500 µg or more, about 3000 µg or more, about 3500 µg or more, about 4000 µg or more, about 4500 µg or more, about 5000 µg or more, about 5500 µg or more, about 6000 µg or more, about 6500 µg or more, about 7000 µg or more, about 7500 µg or more, about 8000 µg or more, about 8500 µg or more, about 9000 µg or more, about 9500 µg or more, about 10 mg or more, about 20 mg or more, about 30 mg or more, about 40 mg or more, about 50 mg or more, about 60 mg or more, about 70 mg or more, about 80 mg or more, about 90 mg or more, about 100 mg or more, about 150 mg or more, about 200 mg or more, about 250 mg or more, about 300 mg or more, about 350 mg or more, about 400 mg or more, about 450 mg or more, about 500 mg or more, about 550 mg or more, about 600 mg or more, about 650 mg or more, about 700 mg or more, about 800 mg or more, about 900 mg or more, or about 1 g or more.

Additionally or alternatively, the amount of compounds of Formula (I) present in the berberine composition may be about 0.01 wt.% to about 95 wt.%, about 0.1 wt.% to about 95 wt.%, about 1 wt.% to about 95 wt.%, about 5 wt.% to about 95 wt.%, about 10 wt.% to about 95 wt.%, about 15 wt.% to about 95 wt.%, about 20 wt.% to about 95 wt.%, about 30 wt.% to about 95 wt.%, about 40 wt.% to about 95 wt.%, about 50 wt.% to about 95 wt.%, about 60 wt.% to about 95 wt.%, about 70 wt.% to about 95 wt.%, about 80 wt.% to about 95 wt.%; about 0.01 wt.% to about 85 wt.%, about 0.1 wt.% to about 85 wt.%, about 1 wt.% to about 85 wt.%, about 5 wt.% to about 85 wt.%, about 10 wt.% to about 85 wt.%, about 15 wt.% to about 85 wt.%, about 20 wt.% to about 85 wt.%, about 30 wt.% to about 85 wt.%, about 40 wt.% to about 85 wt.%, about 50 wt.% to about 85 wt.%, about 60 wt.% to about 85 wt.%, about 70 wt.% to about 85 wt.%; about 0.01 wt.% to about 75 wt.% about 0.1 wt.% to about 75 wt.%, about 1 wt.% to about 75 wt.%, about 5 wt.% to about 75 wt.%, about 10 wt.% to about 75 wt.%, about 15 wt.% to about 75 wt.%, about 20 wt.% to about 75 wt.%, about 30 wt.% to about 75 wt.%, about 40 wt.% to about 75 wt.%, about 50 wt.% to about 75 wt.%, about 60 wt.% to about 75 wt.%; about 0.01 wt.% to about 65 wt.%, about 0.1 wt.% to about 65 wt.%, about 1 wt.% to about 65 wt.%, about 5 wt.% to about 65 wt.%, about 10 wt.% to about 65 wt.%, about 15 wt.% to about 65 wt.%, about 20 wt.% to about 65 wt.%, about 30 wt.% to about 65 wt.%, about 40 wt.% to about 65 wt.%, about 50 wt.% to about 65 wt.%; about 0.01 wt.% to about 55 wt.%, about 0.1 wt.% to about 55 wt.%, about 1 wt.% to about 55 wt.%, about 5 wt.% to about 55 wt.%, about 10 wt.% to about 55 wt.%, about 15 wt.% to about 55 wt.%, about 20 wt.% to about 55 wt.%, about 30 wt.% to about 55 wt.%, about 40 wt.% to about 55 wt.%; about 0.01 wt.% to about 45 wt.%, about 0.1 wt.% to about 45 wt. %, about 1 wt.% to about 45 wt.%, about 5 wt.% to about 45 wt.%, about 10 wt.% to about 45 wt.%, about 15 wt.% to about 45 wt.%, about 20 wt.% to about 45 wt.%, about 30 wt.% to about 45 wt.%; about 0.01 wt.% to about 35 wt.%, about 0.1 wt.% to about 35 wt.%, about 1 wt.% to about 35 wt.%, about 5 wt.% to about 35 wt.%, about 10 wt.% to about 35 wt.%, about 15 wt.% to about 35 wt.%, about 20 wt.% to about 35 wt.%; about 0.01 wt.% to about 25 wt.%, about 0.1 wt.% to about 25 wt.%, about 1 wt.% to about 25 wt.%, about 5 wt.% to about 25 wt.%, about 10 wt.% to about 25 wt.%; about 0.1 wt.% to about 15 wt.%, about 1 wt.% to about 15 wt.%, about 5 wt.% to about 15 wt.%, about 10 wt.% to about 15 wt.%; about 0.01 wt.% to about 25 wt.%, about 0.1 wt.% to about 10 wt.%, about 1 wt.% to about 10 wt.%, or about 5 wt.% to about 10 wt.%, including ranges and subranges thereof, based on the total weight of the berberine composition.

The berberine compositions may further comprise at least one tetrahydroprotoberberine, tetrahydropalmatine, stepholidine, or a combination of two or more thereof. In one embodiment, the tetrahydropalmatine is *I*-tetrahydropalmatine. In another embodiment, the stepholidine is *I*-stepholidine. The berberine compositions may include tetrahydroprotoberberine, tetrahydropalmatine, stepholidine, or a combination thereof in an amount ranging from about 0.1 wt.% to about 95 wt.%, based on the total weight of the berberine compositions. For instance, the berberine composition may include tetrahydroprotoberberine, tetrahydropalmatine, stepholidine, or a combination thereof in an amount of about 0.01 wt.% to about 95 wt.%, about 0.1 wt.% to about 95 wt.%, about 1 wt.% to about 95 wt.%, about 5 wt.% to about 95 wt.%, about 10 wt.% to about 95 wt.%, about 15 wt.% to about 95 wt.%, about 20 wt.% to about 95 wt.%, about 30 wt.% to about 95 wt.%, about 40 wt.% to about 95 wt.%, about 50 wt.% to about 95 wt.%, about 60 wt.% to about 95 wt.%, about 70 wt.% to about 95 wt.%, about 80 wt.% to about 95 wt.%; about 0.01 wt.% to about 85 wt.%, about 0.1 wt.% to about 85 wt.%, about 1 wt.% to about 85 wt.%, about 5 wt.% to about 85 wt.%, about 10 wt.% to about 85 wt.%, about 15 wt.% to about 85 wt.%, about 20 wt.% to about 85 wt.%, about 30 wt.% to about 85 wt.%, about 40 wt.% to about 85 wt.%, about 50 wt.% to about 85 wt.%, about 60 wt.% to about 85 wt.%, about 70 wt.% to about 85 wt.%; about 0.01 wt.% to about 75 wt.%, about 0.1 wt.% to about 75 wt.%, about 1 wt.% to about 75 wt.%, about 5 wt.% to about 75 wt.%, about 10 wt.% to about 75 wt.%, about 15 wt.% to about 75 wt.%, about 20 wt.% to about 75 wt.%, about 30 wt.% to about 75 wt.%, about 40 wt.% to about 75 wt.%, about 50 wt.% to about 75 wt.%, about 60 wt.% to about 75 wt.%; about 0.01 wt.% to about 65 wt.% about 0.1 wt.% to about 65 wt.%, about 1 wt.% to about 65 wt.%, about 5 wt.% to about 65 wt.%, about 10 wt.% to about 65 wt.%, about 15 wt.% to about 65 wt.%, about 20 wt.% to about 65 wt.%, about 30 wt.% to about 65 wt.%, about 40 wt.% to about 65 wt.%, about 50 wt.% to about 65 wt.%; about 0.01 wt.% to about 55 wt.%, about 0.1 wt.% to about 55 wt.%, about 1 wt.% to about 55 wt.%, about 5 wt.% to about 55 wt.%, about 10 wt.% to about 55 wt.%, about 15 wt.% to about 55 wt.%, about 20 wt.% to about 55 wt.%, about 30 wt.% to about 55 wt.%, about 40 wt.% to about 55 wt.%; about 0.01 wt.% to about 45 wt.%, about 0.1 wt.% to about 45 wt.%, about 1 wt.% to about 45 wt.%, about 5 wt.% to about 45 wt.%, about 10 wt.% to about 45 wt.%, about 15 wt.% to about 45 wt.%, about 20 wt.% to about 45 wt.%, about 30 wt.% to about 45 wt.%; about 0.01 wt.% to about 35 wt.%, about 0.1 wt.% to about 35 wt.%, about 1 wt.% to about 35 wt.%, about 5 wt.% to about 35 wt.%, about 10 wt.% to about 35 wt.%, about 15 wt.% to about 35 wt.%, about 20 wt.% to about 35 wt.%; about 0.01 wt.% to about 25 wt.%, about 0.1 wt.% to about 25 wt.%, about 1 wt.% to about 25 wt.%, about 5 wt.% to about 25 wt.%, about 10 wt.% to about 25 wt.%; about 0.01 wt.% to about 15 wt.% about 0.1 wt.% to about 15 wt.%, about 1 wt.% to about 15 wt.%, about 5 wt.% to about 15 wt.%, about 10 wt.% to about 15 wt.%; about 0.01 wt.% to about 10 wt.% about 0.1 wt.% to about 10 wt.%, about 1 wt.% to about 10 wt.%, or about 5 wt.% to about 10 wt.%, including ranges and subranges thereof, based on the total weight of the berberine composition.

Additionally or alternatively, the berberine compositions may be formulated to have a weight ratio of at least one compound (e.g., compounds having a structure according to Formula (VI)) of Formula (I) to tetrahydroprotoberberine, tetrahydropalmatine, stepholidine, or a combination thereof of 1:100 to 100:1, 1:50 to 50:1, 1:20 to 20:1 1:10 to 10:1, 1:9 to 10:1, 1:8 to 10:1, 1:7 to 10:1, 1:6 to 10:1, 1:5 to 10:1, 1:4 to 10:1, 1:3 to 10:1, 1:2 to 10:1, 1:1 to 10:1, 1:10 to 9:1, 1:10 to 8:1, 1:10 to 7:1, 1:10 to 6:1, 1:10 to 5:1, 1:10 to 4:1, 1:10 to 3:1, 1:10 to 2:1, or 1:10 to 1:1, including ranges and subranges thereof. In one instance, the weight ratio of the total weight of compounds of Formula (I) to the total weight of tetrahydroprotoberberine, tetrahydropalmatine, and stepholidine is 1:10 to 10:1, 1:9 to 10:1, 1:8 to 10:1, 1:7 to 10:1, 1:6 to 10:1, 1:5 to 10:1, 1:4 to 10:1, 1:3 to 10:1, 1:2 to 10:1, 1:1 to 10:1, 1:10 to 9:1, 1:10 to 8:1, 1:10 to 7:1, 1:10 to 6:1, 1:10 to 5:1, 1:10 to 4:1, 1:10 to 3:1, 1:10 to 2:1, or 1:10 to 1:1, including ranges and subranges thereof. One of ordinary skill would be able to prepare the compounds of Formula (I) using known methods and/or in the art in view of the disclosure herein.

The berberine compositions may further comprise at least one excipient. Suitable excipients include pharmaceutically acceptable excipients, such as diluents, binders, fillers, buffering agents, pH modifying agents, disintegrants, dispersants, preservatives, lubricants, taste-masking agents, flavoring agents, coloring agents, or combinations thereof. The amount and types of excipients utilized to form pharmaceutical compositions may be selected according to known principles of pharmaceutical science.

In one embodiment, the excipient may be a diluent. The diluent may be compressible (i.e., plastically deformable) or abrasively brittle. Non-limiting examples of suitable compressible diluents include microcrystalline cellulose (MCC), cellulose derivatives, cellulose powder, cellulose esters (i.e., acetate and butyrate mixed esters), ethyl cellulose, methyl cellulose, hydroxypropyl cellulose, hydroxypropyl methylcellulose, sodium carboxymethylcellulose, corn starch, phosphated corn starch, pregelatinized corn starch, rice starch, potato starch, tapioca starch, starch-lactose, starch-calcium carbonate, sodium starch glycolate, glucose, fructose, lactose, lactose monohydrate, sucrose, xylose, lactitol, mannitol, malitol, sorbitol, xylitol, maltodextrin, and trehalose. Non-limiting examples of suitable abrasively brittle diluents include dibasic calcium phosphate (anhydrous or dihydrate), calcium phosphate tribasic, calcium carbonate, and magnesium carbonate.

In another embodiment, the excipient may be a binder. Suitable binders include, but are not limited to, starches, pregelatinized starches, gelatin, polyvinylpyrrolidone, cellulose, methylcellulose, sodium carboxymethylcellulose, ethylcellulose, polyacrylamides, polyvinyloxoazolidone, polyvinylalcohols, C₁₂-C₁₈ fatty acid alcohol, polyethylene glycol, polyols, saccharides

In another embodiment, the excipient may be a filler. Suitable fillers include, but are not limited to, carbohydrates, inorganic compounds, and polyvinylpyrrolidone. By way of non-limiting example, the filler may be calcium sulfate, both di- and tri-basic, starch, calcium carbonate, magnesium carbonate, microcrystalline cellulose, dibasic calcium phosphate, magnesium carbonate, magnesium oxide, calcium silicate, talc, modified starches, lactose, sucrose, mannitol, or sorbitol.

In still another embodiment, the excipient may be a buffering agent. Representative examples of suitable buffering agents include, but are not limited to, phosphates, carbonates, citrates, tris buffers, and buffered saline salts (e.g., Tris buffered saline or phosphate buffered saline).

In various embodiments, the excipient may be a pH modifier. By way of non-limiting example, the pH modifying agent may be sodium carbonate, sodium bicarbonate, sodium citrate, citric acid, or phosphoric acid.

In a further embodiment, the excipient may be a disintegrant. The disintegrant may be non-effervescent or effervescent. Suitable examples of non-effervescent disintegrants include, but are not limited to, starches such as corn starch, potato starch, pregelatinized and modified starches thereof, sweeteners, clays, such as bentonite, micro-crystalline cellulose, alginates, sodium starch glycolate, gums such as agar, guar, locust bean, karaya, pecitin, and tragacanth. Non-limiting examples of suitable effervescent disintegrants include sodium bicarbonate in combination with citric acid and sodium bicarbonate in combination with tartaric acid.

In yet another embodiment, the excipient may be a dispersant or dispersing enhancing agent. Suitable dispersants may include, but are not limited to, starch, alginic acid, polyvinylpyrrolidones, guar gum, kaolin, bentonite, purified wood cellulose, sodium starch glycolate, isoamorphous silicate, and microcrystalline cellulose.

In another alternate embodiment, the excipient may be a preservative. Non-limiting examples of suitable preservatives include antioxidants, such as BHA, BHT, vitamin A, vitamin C, vitamin E, or retinyl palmitate, citric acid, sodium citrate; chelators such as EDTA or EGTA; and antimicrobials, such as parabens, chlorobutanol, or phenol.

In a further embodiment, the excipient may be a lubricant. Non-limiting examples of suitable lubricants include minerals such as talc or silica; and fats such as vegetable stearin, magnesium stearate, or stearic acid.

In yet another embodiment, the excipient may be a taste-masking agent. Taste-masking materials include cellulose ethers; polyethylene glycols; polyvinyl alcohol; polyvinyl alcohol and polyethylene glycol copolymers; monoglycerides or triglycerides; acrylic polymers; mixtures of acrylic polymers with cellulose ethers; cellulose acetate phthalate; and combinations thereof.

In an alternate embodiment, the excipient may be a flavoring agent. Flavoring agents may be chosen from synthetic flavor oils and flavoring aromatics and/or natural oils, extracts from plants, leaves, flowers, fruits, and combinations thereof.

In still a further embodiment, the excipient may be a coloring agent. Suitable color additives include, but are not limited to, food, drug and cosmetic colors (FD&C), drug and cosmetic colors (D&C), or external drug and cosmetic colors (Ext. D&C).

The weight fraction of the excipient or combination of excipients in the composition may be about 99% or less, about 97% or less, about 95% or less, about 90% or less, about 85% or less, about 80% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, about 5% or less, about 2% or less, or about 1% or less of the total weight of the berberine composition.

The berberine composition may be formulated into various dosage forms and administered by a number of different means that will deliver a therapeutically effective amount of the active ingredient. Such compositions may be administered orally, parenterally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term, "parenteral," as used herein includes subcutaneous, intravenous, intramuscular, or intrasternal injection, or infusion techniques. Formulation of drugs is discussed in, for example, Gennaro, A. R., Remington's Pharmaceutical Sciences, Mack Publishing Co., Easton, Pa. (18th ed, 1995), and Liberman, H. A. and Lachman, L., Eds., Pharmaceutical Dosage Forms, Marcel Dekker Inc., New York, N.Y. (1980). In a specific embodiment, the berberine composition may be a food supplement or a cosmetic.

Solid dosage forms for oral administration may be contained in capsules, tablets, caplets, pills, powders, pellets, and granules. In such solid dosage forms, the active ingredient is ordinarily combined with one or more pharmaceutically acceptable excipients, examples of which are detailed above. Oral preparations may also be administered as aqueous suspensions, elixirs, or syrups. For these, the active ingredient may be combined with various sweetening or flavoring agents, coloring agents, and, if so desired, emulsifying and/or suspending agents, as well as diluents such as water, ethanol, glycerin, and combinations thereof.

For parenteral administration (including subcutaneous, intradermal, intravenous, intramuscular, and intraperitoneal), the preparation may be an aqueous or an oil-based solution. Aqueous solutions may include a sterile diluent such as water, saline solution, a pharmaceutically acceptable polyol such as glycerol, propylene glycol, or other synthetic solvents; an antibacterial and/or antifungal agent such as benzyl alcohol, methyl paraben, chlorobutanol, phenol, thimerosal, and the like; an antioxidant such as ascorbic acid or sodium bisulfite; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. The pH of the aqueous solution may be adjusted with acids or bases such as hydrochloric acid or sodium hydroxide. Oil-based solutions or suspensions may further comprise sesame, peanut, olive oil, or mineral oil. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules, and tablets.

For topical (e.g., transdermal or transmucosal) administration, penetrants appropriate to the barrier to be permeated are generally included in the preparation. Pharmaceutical berberine compositions adapted for topical administration may be formulated as ointments, creams, suspensions, lotions, powders, solutions, pastes, gels, sprays, aerosols, or oils. In some embodiments, the pharmaceutical berberine composition is applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical berberine compositions adapted for topical administration to the eye include eye drops wherein the active ingredient is dissolved or suspended in a suitable carrier, especially an aqueous solvent. Pharmaceutical berberine compositions adapted for topical administration in the mouth include lozenges, pastilles, and mouth washes. Transmucosal administration may be accomplished through the use of nasal sprays, aerosol sprays, tablets, or suppositories, and transdermal administration may be via ointments, salves, gels, patches, suspensions, or creams as generally known in the art.

In certain embodiments, a berberine composition comprising Formula (I) or a salt of the compound of Formula (I) is encapsulated in a suitable vehicle to either aid in the delivery of the compound of Formula (I) to target cells, to increase the stability of the composition, or to minimize potential toxicity of the composition. As will be appreciated by a skilled artisan, a variety of vehicles are suitable for delivering a composition of the present disclosure. Non-limiting examples of structured fluid delivery systems may include nanoparticles, liposomes, microemulsions, micelles, dendrimers, and other phospholipid-containing systems. Methods of incorporating compositions into delivery vehicles are known in the art.

In one alternative embodiment, a liposome delivery vehicle may be utilized. Liposomes, depending upon the embodiment, may be used for delivery of a berberine composition comprising compounds of Formula (I) or a salt of the compound of Formula (I) in view of their structural and chemical properties. Generally, liposomes are spherical vesicles with a phospholipid bilayer membrane. The lipid bilayer of a liposome may fuse with other bilayers (e.g., the cell membrane), thus delivering the contents of the liposome to cells.

Liposomes may be comprised of a variety of different types of phosolipids having varying hydrocarbon chain lengths. Phospholipids generally comprise two fatty acids linked through glycerol phosphate to one of a variety of polar groups. Suitable phospholids include phosphatidic acid (PA), phosphatidylserine (PS), phosphatidylinositol (PI), phosphatidylglycerol (PG), diphosphatidylglycerol (DPG), phosphatidylcholine (PC), and phosphatidylethanolamine (PE). The fatty acid chains comprising the phospholipids may range from about 6 to about 26 carbon atoms in length, and the lipid chains may be saturated or unsaturated. Suitable fatty acid chains include (common name presented in parentheses) n-dodecanoate (laurate), n-tretradecanoate (myristate), n-hexadecanoate (palmitate), n-octadecanoate (stearate), n-eicosanoate (arachidate), n-docosanoate (behenate), n-tetracosanoate (lignocerate), cis-9-hexadecenoate (palmitoleate), cis-9-octadecanoate (oleate), cis,cis-9,12-octadecandienoate (linoleate), all cis-9, 12, 15-octadecatrienoate (linolenate), and all cis-5,8,11,14-eicosatetraenoate (arachidonate). The two fatty acid chains of a phospholipid may be identical or different. Acceptable phospholipids include dioleoyl PS, dioleoyl PC, distearoyl PS, distearoyl PC, dimyristoyl PS, dimyristoyl PC, dipalmitoyl PG, stearoyl, oleoyl PS, palmitoyl, linolenyl PS, and the like.

The phospholipids may come from any natural source, and, as such, may comprise a mixture of phospholipids. For example, egg yolk is rich in PC, PG, and PE, soy beans contains PC, PE, PI, and PA, and animal brain or spinal cord is enriched in PS. Phospholipids may come from synthetic sources too. Mixtures of phospholipids having a varied ratio of individual phospholipids may be used. Mixtures of different phospholipids may result in liposome compositions having advantageous activity or stability of activity properties. The above mentioned phospholipids may be mixed, in optimal ratios with cationic lipids, such as N-(1-(2,3-dioleolyoxy)propyl)-N,N,N-trimethyl ammonium chloride, 1,1'-dioctadecyl-3,3,3',3'-tetramethylindocarbocyanine perchloarate, 3,3'-deheptyloxacarbocyanine iodide, 1,1'-dedodecyl-3,3,3',3'-tetramethylindocarbocyanine perchloarate, 1,1'-dioleyl-3,3,3',3'-tetramethylindo carbocyanine methanesulfonate, N-4-(delinoleylaminostyryl)-N-methylpyridinium iodide, or 1,1,-dilinoleyl-3,3,3',3'-tetramethylindocarbocyanine perchloarate.

Liposomes may optionally comprise sphingolipids, in which spingosine is the structural counterpart of glycerol and one of the one fatty acids of a phosphoglyceride, or cholesterol, a major component of animal cell membranes. Liposomes may optionally contain pegylated lipids, which are lipids covalently linked to polymers of polyethylene glycol (PEG). PEGs may range in size from about 500 to about 10,000 daltons.

Liposomes may further comprise a suitable solvent. The solvent may be an organic solvent or an inorganic solvent. Suitable solvents include, but are not limited to, dimethylsulfoxide (DMSO), methylpyrrolidone, N-methylpyrrolidone, acetronitrile, alcohols, dimethylformamide, tetrahydrofuran, or combinations thereof.

Liposomes carrying a composition comprising the compound of Formula (I) may be prepared by any known method of preparing liposomes for drug delivery, such as, for example, detailed in U.S. Pat. Nos. 4,241,046, 4,394,448, 4,529,561, 4,755,388, 4,828,837, 4,925,661, 4,954,345, 4,957,735, 5,043,164, 5,064,655, 5,077,211, and 5,264,618. For example, liposomes may be prepared by sonicating lipids in an aqueous solution, solvent injection, lipid hydration, reverse evaporation, or freeze drying by repeated freezing and thawing. In a preferred embodiment the liposomes are formed by sonication. The liposomes may be multilamellar, which have many layers like an onion, or unilamellar. The liposomes may be large or small. Continued high-shear sonication tends to form smaller unilamellar lipsomes.

As would be apparent to one of ordinary skill, all of the parameters that govern liposome formation may be varied. These parameters include, but are not limited to, temperature, pH, concentration of methionine compound, concentration, and composition of lipid, concentration of multivalent cations, rate of mixing, presence of and concentration of solvent.

The berberine composition may be formulated as part of a microemulsion. Microemulsions are generally clear, thermodynamically stable solutions comprising an aqueous solution, a surfactant, and "oil." The "oil" in this case, is the supercritical fluid phase. The surfactant rests at the oil-water interface. Any of a variety of surfactants are suitable for use in microemulsion formulations including those described herein or otherwise known in the art. The aqueous microdomains suitable for use in the disclosure generally will have characteristic structural dimensions from about 5 nm to about 100 nm. Aggregates of this size are poor scatterers of visible light and hence, these solutions are optically clear. As will be appreciated by a skilled artisan, microemulsions can and will have a multitude of different microscopic structures including sphere, rod, or disc shaped aggregates. In one embodiment, the structure may be micelles, which are the simplest microemulsion structures that are generally spherical or cylindrical objects. Micelles are like drops of oil in water, and reverse micelles are like drops of water in oil. In an alternative embodiment, the microemulsion structure is the lamellae. It comprises consecutive layers of water and oil separated by layers of surfactant. The "oil" of microemulsions optimally comprises phospholipids. Any of the phospholipids detailed above for liposomes are suitable for embodiments directed to microemulsions. A composition comprising at least one anti-viral therapeutic derivative may be encapsulated in a microemulsion by any method generally known in the art.

In yet another embodiment, the berberine composition comprising compounds of Formula (I) or a salt thereof may be delivered in a dendritic macromolecule, or a dendrimer. Generally, a dendrimer is a branched tree-like molecule, in which each branch is an interlinked chain of molecules that divides into two new branches (molecules) after a certain length. This branching continues until the branches (molecules) become so densely packed that the canopy forms a globe. Generally, the properties of dendrimers are determined by the functional groups at their surface. For example, hydrophilic end groups, such as carboxyl groups, would typically make a water-soluble dendrimer. Alternatively, phospholipids may be incorporated in the surface of a dendrimer to facilitate absorption across the skin. Any of the phospholipids detailed for use in liposome embodiments are suitable for use in dendrimer embodiments. Any method generally known in the art may be utilized to make dendrimers and to encapsulate compositions of the disclosure therein. For example, dendrimers may be produced by an iterative sequence of reaction steps, in which each additional iteration leads to a higher order dendrimer. Consequently, they have a regular, highly branched 3D structure, with nearly uniform size and shape. Furthermore, the final size of a dendrimer is typically controlled by the number of iterative steps used during synthesis. A variety of dendrimer sizes are suitable for use in the disclosure. Generally, the size of dendrimers may range from about 1 nm to about 100 nm.

Additionally, the present disclosure relates to the compounds for use in methods for administering the berberine compositions comprising compounds of Formula (I) and/or salts thereof. The methods disclosed herein may be used for treating nicotine addiction, reducing nicotine dependency, and/or reducing nicotine urges. Methods of treatment are not covered by the claims and are included in the description for the completeness of the disclosure only. As used herein, terms "treatment", "treating", and the like are used interchangeably herein to generally mean obtaining a desired pharmacological and/or physiological effect. The terms are used in a manner somewhat differently than the terms are typically used in that what is intended by the method of treatment of the disclosure is to allow a patient to reduce their nicotine dependency by reducing their urges for nicotine using the berberine compositions and the compounds of Formula (I) disclosed herein, thereby helping the patient to quit smoking.

The amount of the berberine compositions that is administered to the individual can and will vary depending upon the type or form of the berberine compositions, the individual, and the particular mode of administration. Those skilled in the art will appreciate that dosages may also be determined with guidance from Goodman & Goldman's The Pharmacological Basis of Therapeutics, Tenth Edition (2001), Appendix II, pp. 475-493, and the Physicians' Desk Reference.

The methods of the disclosure may include administering an amount of a berberine composition comprising one or more compounds of Formula (I) topically, orally, or parenterally. For topical administration, the dosage of berberine composition may be in the form of a cream, a serum, a lotion, a gel, or the like. In at least one case, the dosage of the berberine composition contained in the gel of an adhesive gel patch.

For oral administration, the method may include administering an amount of the berberine composition in the form of a solid dosage or a liquid dosage. Solid dosage forms for oral administration include capsules, tablets, caplets, pills, powders, pellets, and granules. In such solid dosage forms, the compounds of Formula (I) are, typically, combined with one or more excipients, such as those described above. Liquid dosages of the berberine composition may be in the form of aqueous suspensions, elixirs, or syrups. For these, the berberine composition may be combined with various sweetening or flavoring agents, coloring agents, and, if so desired, emulsifying and/or suspending agents, as well as diluents such as water, ethanol, glycerin, and combinations thereof.

For parenteral administration, the dosage of berberine composition may be an aqueous solution, an oil-based solution, or in the form of a solid dosage. Aqueous solutions may include a sterile diluent such as water, saline solution, a pharmaceutically acceptable polyol such as glycerol, propylene glycol, or other synthetic solvents; an antibacterial and/or antifungal agent such as benzyl alcohol, methyl paraben, chlorobutanol, phenol, thimerosal, and the like; an antioxidant such as ascorbic acid or sodium bisulfite; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. The pH of the aqueous solution may be adjusted with acids or bases such as hydrochloric acid or sodium hydroxide. Oil-based solutions or suspensions may further comprise sesame, peanut, olive oil, or mineral oil. In some instances, parental administration may be subcutaneous, intravenous, intramuscular, or intrasternal injection, or infusion.

The administration of the compounds of Formula (I) and/or salts thereof may occur on a regular period or an irregular period. For example, the method may include administration of the berberine compositions when a user desires a dosage of the berberine composition, e.g., when a user has a nicotine urge or determines that they are likely to have a nicotine urge soon thereafter. The administration of the berberine compositions may be based on input or determination from a medical practitioner.

In some cases, however, the method may include administering the berberine compositions on a regular period. For example, the method may include administering a predetermined dosage comprising the berberine composition once every hour, once every two hours, once every three hours, once every four hours, once every five hours, once every six hours, once every seven hours, once every eight hours, etc. With some berberine compositions and methods for administration, regular administration may be once every other day, once daily, twice daily, or more than twice daily, or alternatively less frequently than once every other day, such as twice a week, once a week, bi-weekly (every two weeks), once a month, every two months, or once every three months. The overall treatment may be at least two weeks, at least a month, at least 6 months, etc. The treatment may be discontinued as cessation of nicotine addiction stops.

It will be understood that certain types of dosage formulations contemplated herein are well-adapted to less frequent than once daily administration, such as a thin topical film, or transdermal patch that may be adapted for application once monthly, once every other month, or once every 90 days; or a sustained or extended release microparticle formulation adapted for once a month, once every other month, or once every three months administration. The period of time during which the therapeutically effective dosage formulation comprising a therapeutically effective amount of berberine composition is administered can and will vary depending on a number of factors, including the severity of disorder being treated, the type of formulation, route and frequency of administration, the age of the subject, etc. Generally, the period of administration is at least several times a day and/or a week.

In certain embodiments, the methods may include administering a first dosage of the berberine composition on a regular period for a certain amount of time (e.g., two weeks), and subsequently administering a second dosage of the berberine composition on a regular period for a certain amount of time (e.g., two weeks). Preferably, the second dosage contains an amount of the berberine composition that is less than the amount of berberine composition in the first dosage. In some cases, the method may contain a third, a fourth, and/or a fifth dosage, with consecutively lower amounts of berberine composition in each later dosage, to help an individual wean off the berberine composition.

The methods may reduce K_{ATP} channel signaling in an individual. In some cases, the K_{ATP} channel signaling is part of a dopaminergic receptor, an adrenergic receptor, and/or a serotonin receptor. For instance, the methods may include reducing K_{ATP} channel signaling dopaminergic receptor having a D1, D2, D3, or D4 receptor subtype.

As used herein, the term "acyl," alone or as part of another group, denotes the moiety formed by removal of the hydroxy group from the group COOH of an organic carboxylic acid, e.g., RC(O)-, wherein R is R^{X}, R^{X}O-, R^{X}R^{Y}N-, or R^{X}S-, R^{X} is hydrocarbyl, heterosubstituted hydrocarbyl, or heterocyclo, and R^{Y} is hydrogen, hydrocarbyl, or substituted hydrocarbyl.

The term "acyloxy," as used herein, alone or as part of another group, denotes an acyl group as described above bonded through an oxygen linkage (O), e.g., RC(O)O- wherein R is as defined in connection with the term "acyl."

The term "allyl," as used herein, not only refers to compound containing the simple allyl group (CH₂=CH-CH₂-), but also to compounds that contain substituted allyl groups or allyl groups forming part of a ring system.

The term "alkyl," as used herein, describes groups that are preferably lower alkyl containing from one to eight carbon atoms in the principal chain and up to 20 carbon atoms. They may be straight or branched chain and include methyl, ethyl, propyl, isopropyl, butyl, hexyl and the like.

The term "alkenyl," as used herein, describes groups that are preferably lower alkenyl containing from two to eight carbon atoms in the principal chain and up to 20 carbon atoms. They may be straight or branched chain and include ethenyl, propenyl, isopropenyl, butenyl, isobutenyl, hexenyl, and the like.

The term "alkynyl," as used herein, describes groups that are preferably lower alkynyl containing from two to eight carbon atoms in the principal chain and up to 20 carbon atoms. They may be straight or branched chain and include ethynyl, propynyl, butynyl, isobutynyl, hexynyl, and the like.

The term "aromatic," as used herein, alone or as part of another group denotes homo- or heterocyclic conjugated planar ring or ring system comprising delocalized electrons. These aromatic groups are preferably monocyclic (e.g., furan or benzene), bicyclic, or tricyclic groups containing from 5 to 14 atoms in the ring portion. The term "aromatic" encompasses "aryl" groups defined below.

The terms "aryl" or "Ar," as used herein, alone or as part of another group denote homocyclic aromatic groups, preferably monocyclic or bicyclic groups containing from 6 to 10 carbons in the ring portion, such as phenyl, biphenyl, or naphthyl.

The terms "carbocyclo" or "carbocyclic," as used herein, alone or as part of another group denote aromatic or non-aromatic, homocyclic ring or ring system in which all of the atoms in the ring are carbon, with preferably 5 or 6 carbon atoms in each ring. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "halogen" or "halo," as used herein, alone or as part of another group refer to chlorine, bromine, fluorine, and iodine.

The term "heteroatom," as used herein, refers to atoms other than carbon and hydrogen.

The term "heteroaromatic," as used herein, alone or as part of another group denotes aromatic groups having at least one heteroatom in at least one ring, and preferably 5 or 6 atoms in each ring. The heteroaromatic group may have 1 to 4 oxygen, nitrogen, silicone, and/or sulfater atoms (e.g. 1 or 2 oxygen atoms and/or 1 to 4 nitrogen atoms) in the ring, and is bonded to the remainder of the molecule through a carbon. Exemplary groups include furyl, benzofuryl, oxazolyl, isoxazolyl, oxadiazolyl, benzoxazolyl, benzoxadiazolyl, pyrrolyl, pyrazolyl, imidazolyl, triazolyl, tetrazolyl, pyridyl, pyrimidyl, pyrazinyl, pyridazinyl, indolyl, isoindolyl, indolizinyl, benzimidazolyl, indazolyl, benzotriazolyl, tetrazolopyridazinyl, carbazolyl, purinyl, quinolinyl, isoquinolinyl, imidazopyridyl, and the like. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "heterocyclo" or "heterocyclic," as used herein, alone or as part of another group denote optionally substituted, fully saturated or unsaturated, monocyclic or bicyclic, aromatic or non-aromatic groups having at least one heteroatom in at least one ring, and preferably 5 or 6 atoms in each ring. The heterocyclo group may have 1 or 2 oxygen atoms and/or 1 to 4 nitrogen atoms in the ring, and is bonded to the remainder of the molecule through a carbon or heteroatom. Exemplary heterocyclo groups include heteroaromatics as described above. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "hydrocarbon" and "hydrocarbyl," as used herein, refer to organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The compounds described herein may have asymmetric centers. Compounds of the present disclosure containing an asymmetrically substituted atom may be isolated in optically active or racemic form. All chiral, diastereomeric, racemic forms and all geometric isomeric forms of a structure are intended, unless the specific stereochemistry or isomeric form is specifically indicated.

When introducing elements of the embodiments described herein, the articles "a", "an", "the" and "said" are intended to mean that there are one or more of the elements. The terms "comprising", "including" and "having" are intended to be inclusive and mean that there may be additional elements other than the listed elements.

As various changes could be made in the above-described methods without departing from the scope of the disclosure, it is intended that all matter contained in the above description and in the examples given below, shall be interpreted as illustrative.

### Examples

The follow examples are included primarily for enabling skilled artisans to make and use the instant disclosure. Skilled artisans, however, would appreciate that many changes could be made in the disclosure and still obtain a like or similar result without departing from the scope of the disclosure, therefore all matter set forth is to be interpreted as illustrative and not in a limiting sense.

### Example 1. Patch-clamp studies regarding the effect of THPBs on α4β2-nAChR function.

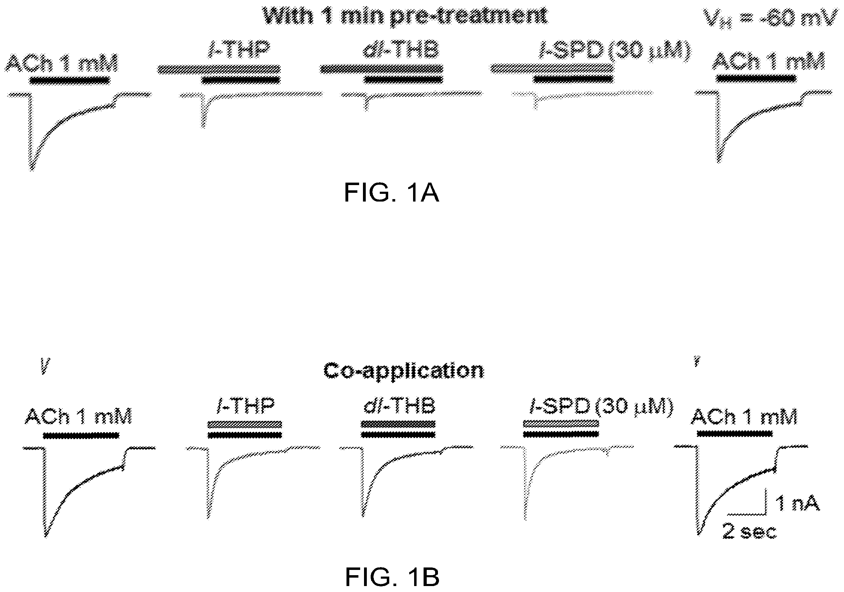

The effects of THPBs on α4β2-nAChR function were evaluated in vitro. Specifically, whole-cell patch-clamp recording technique was employed in α4β2-nAChRs transfected with a human SH-EP1 cell-line. FIG. 1A provides graphs illustrating the effects of a 1 min pre-treatment of 30 µM *I*-THP, *dI*-THB or *I*-SPD on 1 mM ACh-induced current in SH-EP1 cell line. As shown in FIG. 1A, within 1 minute of pretreatment using THPBs (30 µM for each), 1 mM ACh-induced whole-cell currents were dramatically eliminated.

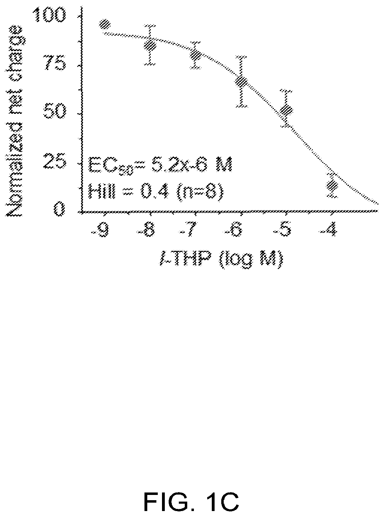

FIG. 1B shows graphs illustrating the effects of co-application of 30 µM *I*-THP, *dI*-THB or *I*-SPD on 1 mM ACh-induced currents in SH-EP1 cells. As seen in FIG. 1B, if THPBs were co-applied with ACh, the ACh-induced currents were also suppressed at the steady-state component, but the peak current component was only mildly reduced. This result suggests that THPBs may have a slow kinetic affinity to ACh binding sites. Thus, when THPBs are co-applied with an agonist, it affects receptor function after agonist opens channels. In this way, THPBs dramatically reduced nAChR current with pre-treatment, but predominantly reduced only steady-state current (not the peak current) when it was co-applied with the agonist. FIG. 1C shows a concentration-dependent inhibition of co-application of *I*-THP on 1 mM ACh-induced currents in SH-EP1 cells (n=8). The IC₅₀ value for current net charge (the area of whole-cell current during agonist exposure divided by Cm) was 5.2x10⁻⁶ M and Hill coefficient was 0.4. These results suggest that THPBs are the antagonists of human α4β2-nAChR.

### Example 2. Biochemical studies on THBs effect human α4β2-nAChR-mediated response.

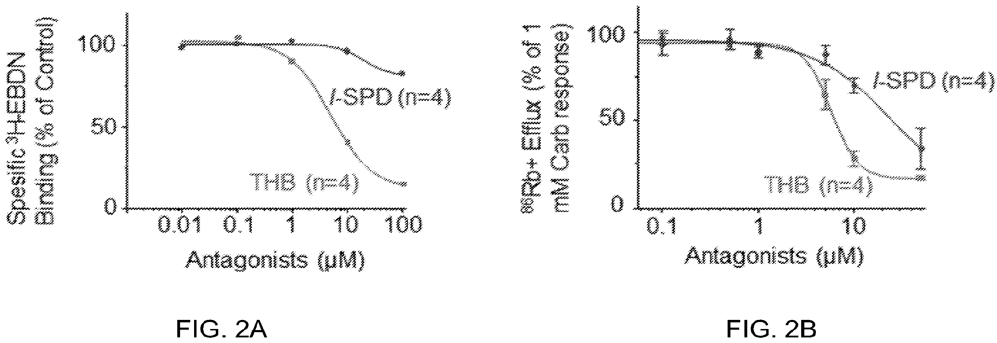

The effect of heterologously expressed α4β2-nAChR resulted in high-affinity, specific binding of ³H-labeled epibatidine (H-EBDN; macroscopic *K*_{D} = 10 pM; *k*ₒₙ = 0.74/min/nM, *k*_{off} = 0.013/min). H-EBDN binding competition studies revealed a high affinity for THB and lower affinity for *I*-SPD (e.g., as seen in FIG. 2A). Heterologously expressed α4β2-nAChR functional studies using ⁶⁶Rb⁺ efflux assays indicate that both THB and *I*-SPD compete to carbachol-induced response, but the THB showed lower IC₅₀ value (7 µM) than *I*-SPD (30 µM) (FIG. 2B). These results demonstrate utility of transfected SH-EP1 cells as models for studies of human α4β2-nAChRs, and THB is likely a potent compound to inhibit human α4β2-nAChR function.

### Example 3. Evaluation of HB on ACh-induced currents between human and rat α4β2-nAChR-mediatedwhole-cell currents.

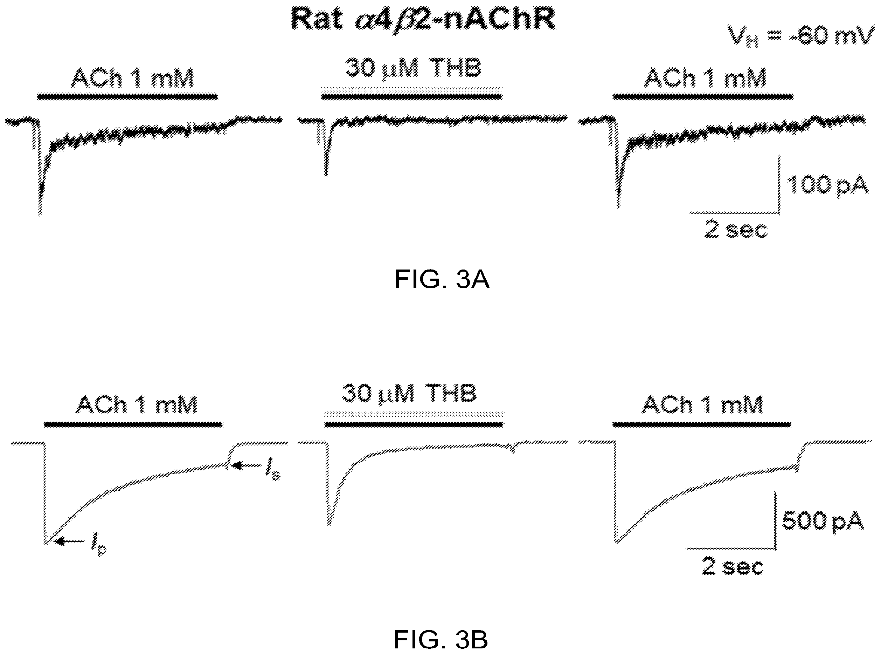

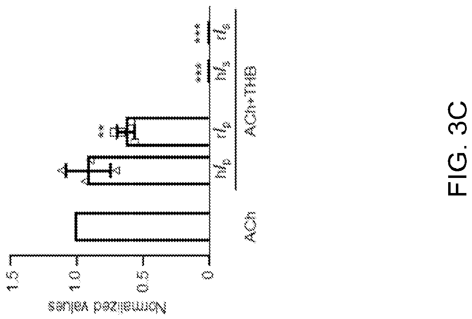

The effects of THB-induced inhibition on the α4β2-nAChR function were evaluating using human α4β2-nAChRs (transfected to SH-EP1 cells) and rat α4β2-nAChRs natively expressed in VTA DA neurons. The 30 µM THB (without pretreatment) inhibited both types (human and rat) of α4β2-nAChR function, and compared to peak current, the steady-state current portion was more sensitive to THB (FIGS. 3A-3C).

### Example 4. Evaluation of the effect of concentration on THB inhibiting α4β2-nAChR-mediated current.

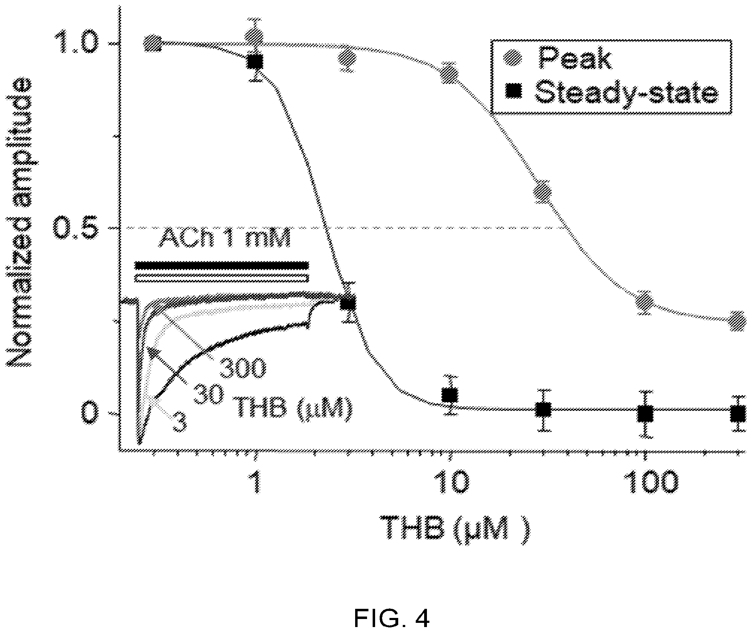

The effect of different concentrations of THB on 1 mM ACh-induced whole-cell currents were further examined in human α4β2-nAChRs in SH-EP1 cells. It was found that THB inhibited the ACh-induced currents, which were reduced with increasing concentrations of THB (see, *e.g.,* FIG. 4). The steady-state current was more sensitive to THB than the peak current. The IC₅₀ values of *I*ₚ and *I*ₛ were 2.2 and 37 µM, respectively.

### Example 5. Evaluation of the effect of THB on nicotine-induced increase in excitatory postsynaptic synaptic currents in VTA DA neurons.

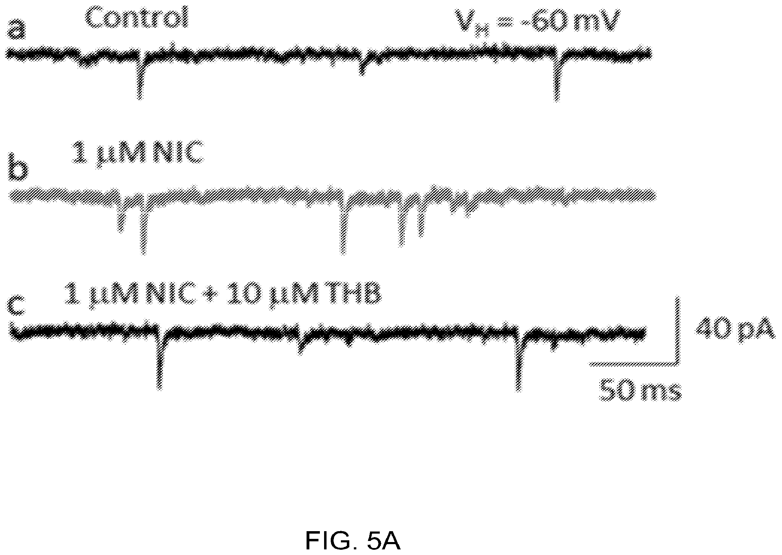

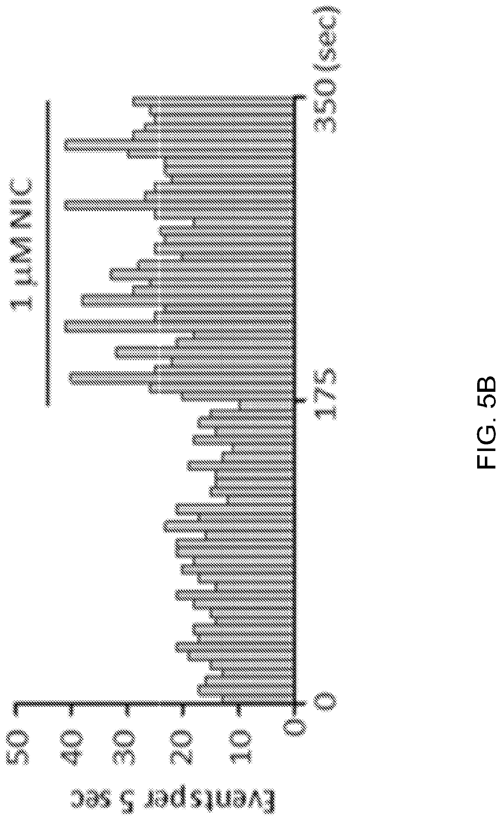

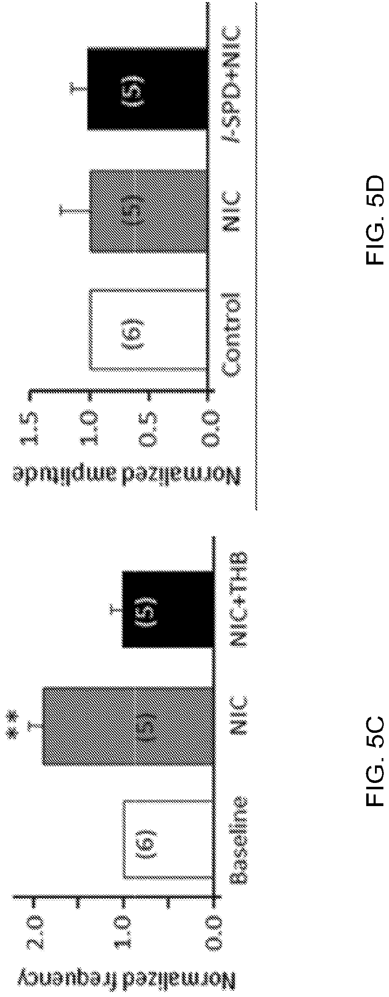

It is known that nicotine increases EPSCs in VTA DA neurons, which is an important mechanism for nicotine-induced synaptic plasticity and neuronal adaptation in mesolimbic circuit. Thus, the effect of *I*-THP on this nicotine-induced EPSC enhancement was studied. FIG. 4 shows the miniature EPSCs in a VTA DA neuron recorded by patch-clamp whole-cell recording in a VTA slice (FIG. 3A). Bath application of 1 µM nicotine significantly increased mEPSC frequency (FIGS. 5A-C), but not amplitude (see, FIG. 5D). Specifically, FIG. 5A shows typical traces of mEPSCs before (a), during NIC exposure (b), and during THB plus NIC co-exposure (c). Co-application of 10 µM *I*-THP abolished nicotine-induced enhancement of mEPSCs (see, FIGS. 5A and 5C). These results suggest that THB is able to reduce nicotine-induced presynaptic glutamate release in VTA DA neurons.

### Example 6. In vivo study of THB on VTA DA neuronal firing in anesthetized rats.

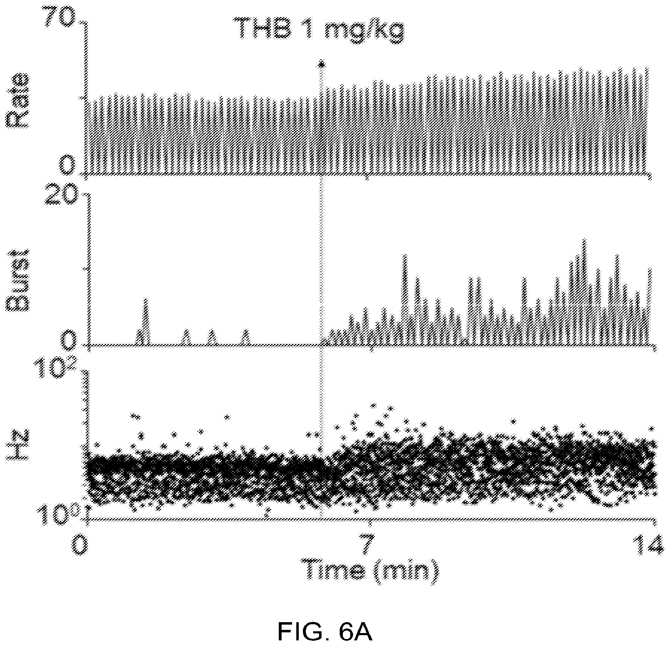

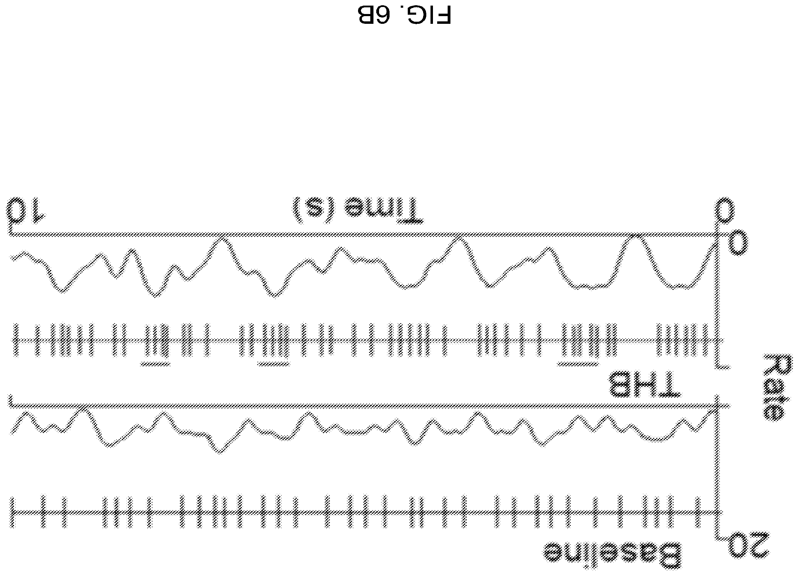

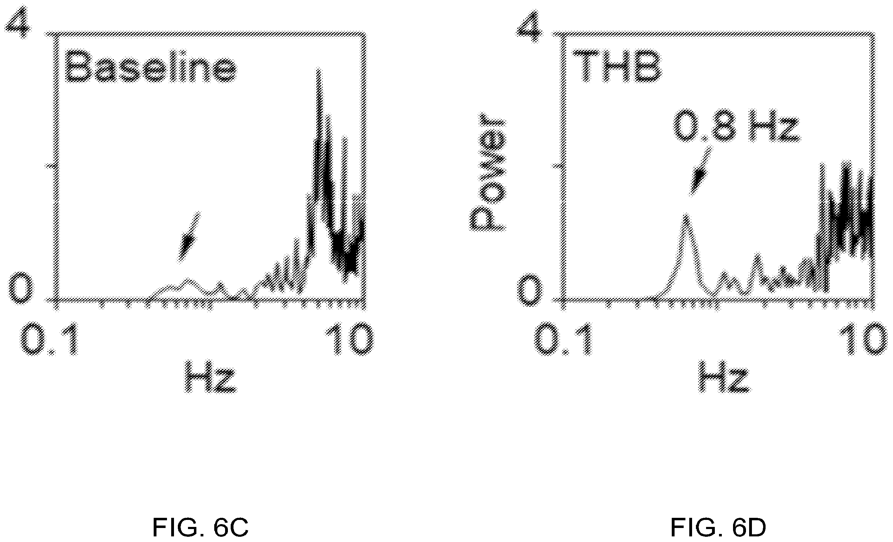

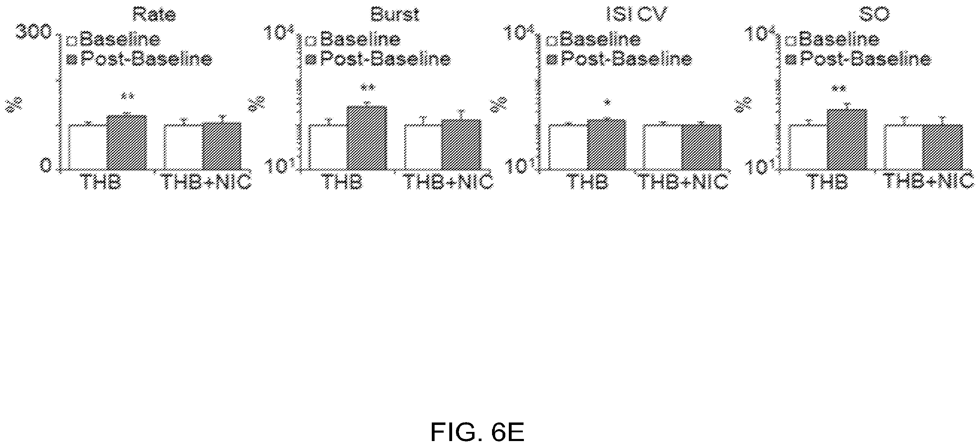

It was tested whether systemic injection of THB affected VTA DA neuron firing. As shown in FIGS. 6A-6C, injection of THB (1 mg/kg, i.v.) increased VTA DA neuron firing rate, bursting fraction (FIG. 6A), and slow oscillations (FIGS. 6B-6D). However, after injection of THB for 5 min, systemic injection of nicotine (0.5 mg/kg, i.v.) failed to increase VTA DA firing rate and bursting fraction. A statistical analysis summary is provided in FIG. 6E. Without being limited to any specific theory, these results suggest that THB itself enhances VTA DA neuron firing perhaps through its blockade of D2 autoreceptors on VTA DA neurons and also activation of cortical 5-TH receptor. THB appears to eliminate nicotinic effect via its blockade of nAChRs. Therefore, the actions of THB are consistent with the double target hypothesis that THB blocks nAChRs (especially α4β2-nAChRs) and enhances DA release from VTA to DA neuronal targets.

### Example 7. Animal behavioral study of I-THP counteracting nicotine-induced CPP.

A conventional biased CPP was performed on male C57BL/6 mice. Mice were put into two-compartment CPP apparatus (Med Associate Inc. VT) for accommodation once (20 min) a day for 3 days and for monitoring the preference to any specific side of compartment. Mice were divided into 3 groups (5 mice per group) and received 2 training sessions per day for 4 days.

The first group of mice (the nicotine group) received either nicotine (1.5 mg/Kg) or saline injection. When the mouse received nicotine, the mouse was placed into the least preferred side for 20 min. When the mouse received a saline injection, it was placed into the opposite side for 20 min. The second group of mice (the I-THP + nicotine group) received a nicotine injection and were placed into the least preferred side for 20 min. Alternatively, the mice of the second group received saline injection and placed into the opposite side. The third group of mice (the I-THP only group) received *I*-THP i.p. injection (10 mg/Kg) and were placed into the least preferred side for 20 min. Alternatively, the mice received a saline injection and were placed into the opposite side.

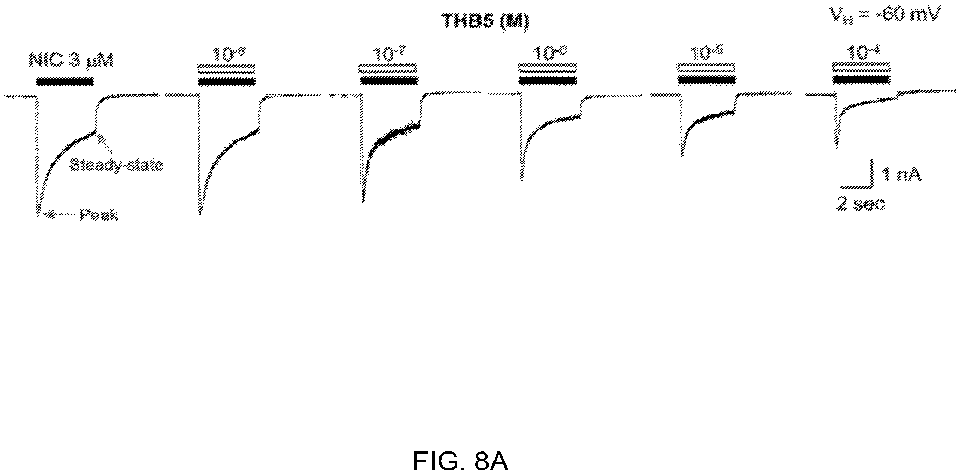

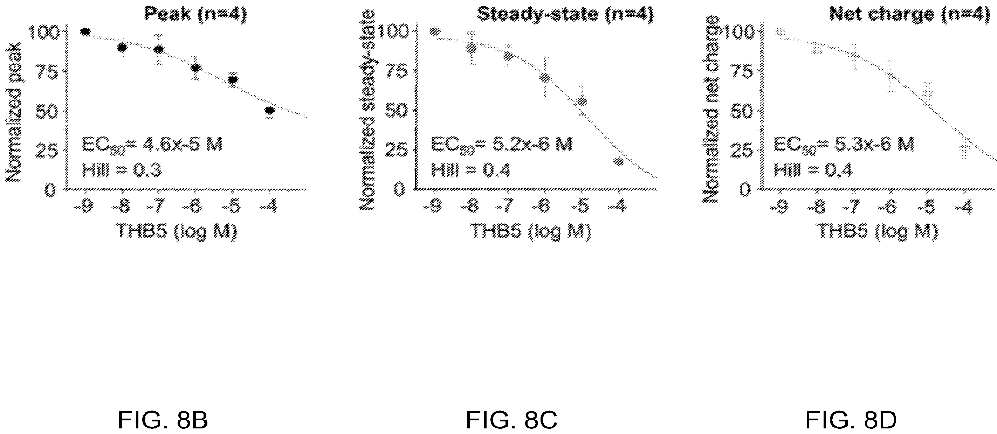

On the 5th day, mice were tested for place preference by allowing free access to both conditioning compartments for 20 minutes. The foregoing 4-day training session was repeated on the 6th day. Mice of group 1 received their treatments without change. Mice of group 2 mice were treated with *I*-THP (*i.p.* injection 10 mg/Kg) and 5 min later given a nicotine injection. After the nicotine injection, the mice of group 2 were placed into the least preferred chamber for 20 min. Alternatively, the mice of group 2 received a saline injection and then received a saline injection 5 min later. After the saline injection, the mice of group 2 were placed into the opposite chamber for 4 days. On the 10th day, the mice were tested for place preference for 20 min (See, Fig 8). These results suggest that nicotine induced significant CPP and *I*-THP counteracts the nicotine rewarding effects after CPP has been established.

### Example 8. The effect of a compound of Formula (I) on human α4β2-nAChR function in SH-EP1 cells.

A compound of formula (I) was producing having a structure according to Formula (VI), below.

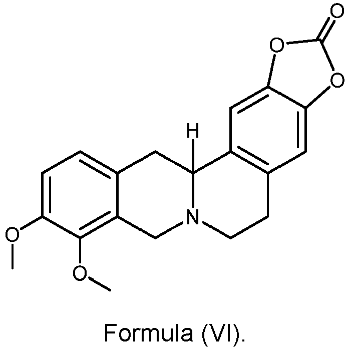

The compounds of Formula (VI) (hereafter referred to "THB6") had a chemical formula of C₂₀H₁₉NO₅, a molecular weight of 353.37, tPSA of 57.23, CLogP of 2.392, and LogS of -3.905. FIG. 8A shows typical traces illustrating the changes of 3 µM nicotine (NIC, about the EC₅₀ concentration)-induced currents with co-application of THB6 with different concentrations. FIGS. 8B-8D provide graphs of concentration-inhibition efficacy curves by measuring the peak current (see FIG. 8B), the steady-state current (FIG. 8C) and the net charge (current response area/Cm) (see FIG. 8D).

### Example 9. The effect of THB6 on human α4β2-nAChR-mediatedcurrents in SH-EP1 cells with and without pre-treatment.

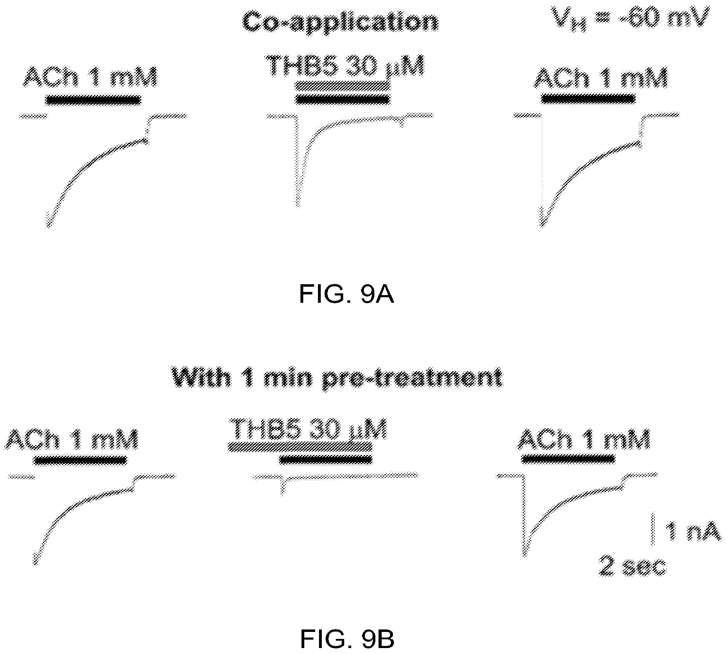

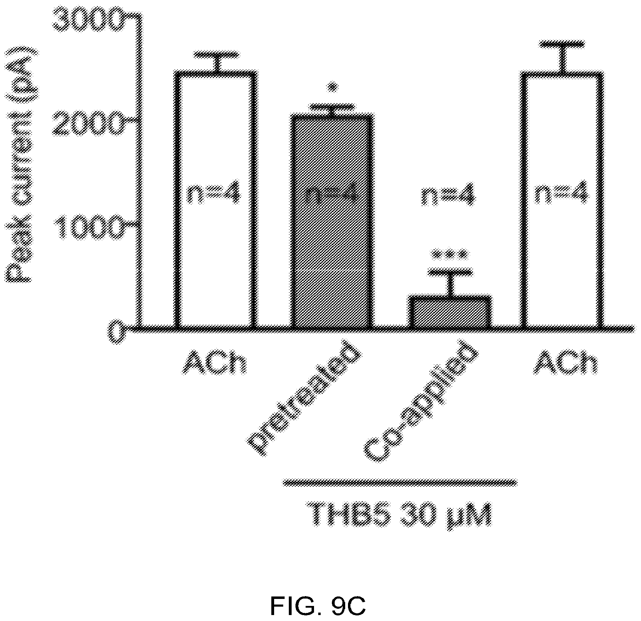

A dosage containing 30 µM THB6 compound was evaluated on human α4β2-nAChR-mediated currents in SH-EP1 cells with and without pre-treatment, similar to Example 2. FIG. 9A provides a graph of traces of co-application of THB6 on ACh-induced current. FIG. 9B provides a graph of traces of the effect of 1 min pretreatment of THB6 on ACh-induced current. FIG. 9C provides a bar graph showing the statistic result of THB6 inhibiting the peak compound of ACh-induced currents. Each bar of the bar graph presented in FIG. 9C represents the mean from 4 cells and vertical bars indicate +Se. A "*" was given to indicate p<0.05, while a "***" was given to indicate p<0.001 in FIG. 9C. Data was analyzed with One-Way ANOVA.

### Example 10. The effect of THB6 on human and mouse α4β2-nAChR-mediated currents.

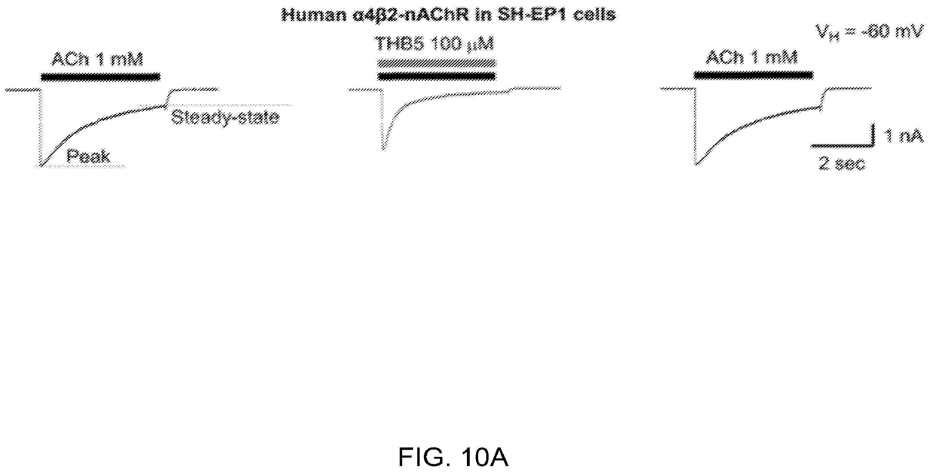

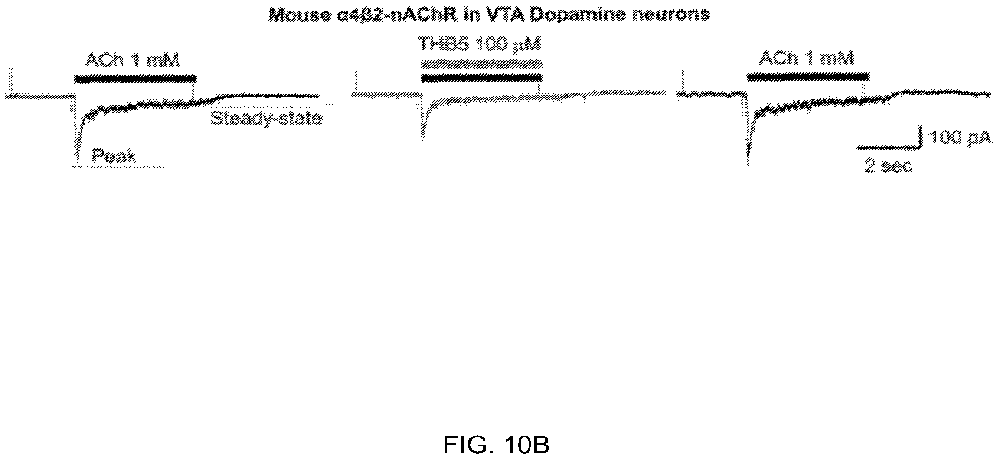

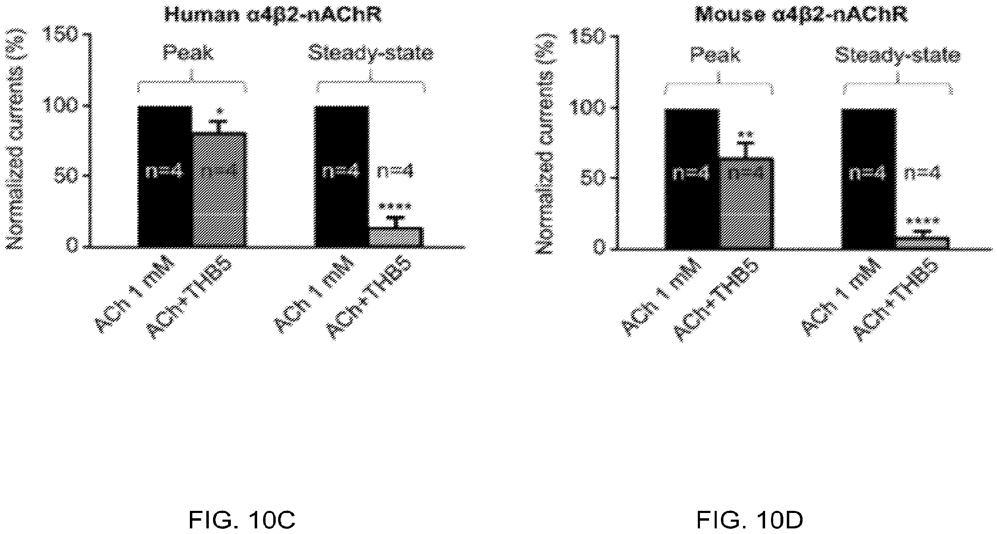

A dosage containing 100 µM THB6 compound was evaluated on human and mouse α4β2-nAChR-mediated currents. FIG. 10A provides a graph of traces of ACh-induced whole-cell currents in human α4β2-nAChRs transfected in human SH-EP1 cells. FIG. 10B provides a graph of traces of ACh-induced whole-cell currents in mouse α4β2-nAChRs transfected in VTA dopamine neurons. As seen in FIG. 10B, the traces show the changes before (left), during (middle) and after (right) the co-application of NIC and THB6 on ACh-induced currents. FIGS. 10C and 10D provide bar graphs showing the statistic result that THB6 inhibits both peak and steady-state compounds of ACh-induced currents. Each bar in the bar graph of FIG. 10C represents the mean from 4 cells and vertical bars indicate +SE. A "*" was given to indicate p<0.05. A "***" was given to indicate p<0.001 in FIG. 9C. Data was analyzed with One-Way ANOVA.

### Example 11. The effect of THB6 on nicotine-induced firing rates.

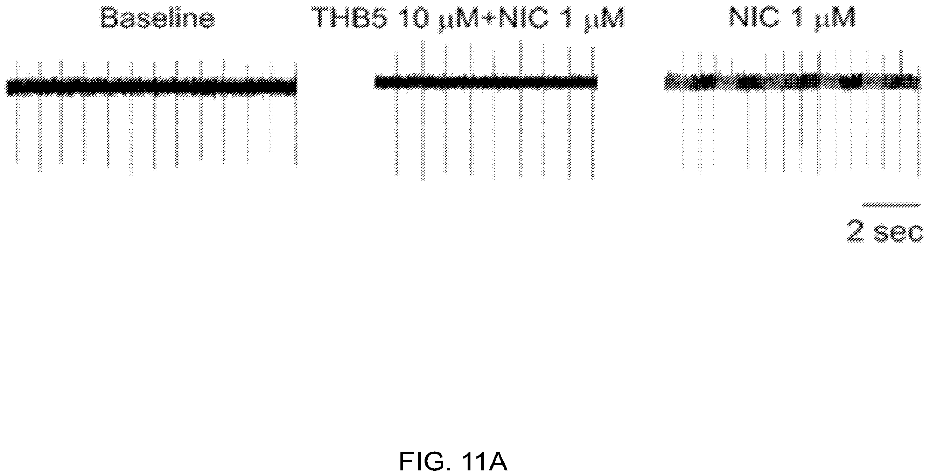

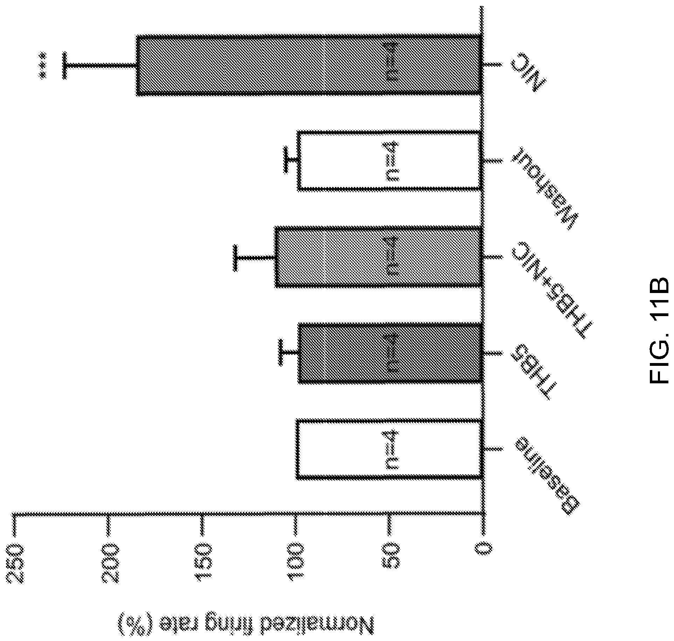

Based on the evaluation of a dosage containing THB6 compounds on VTA slices, it was determined that THB6 prevents nicotine-induced firing rate increase in VTA DA neurons. FIG. 11A is a graph of traces of extracellular action potential (AP) firing using cell-attached patch recording mode in mouse VTA neurons, and showed that THB6 alone did not affect DA neuronal firing, but prevented nicotine-induced firing increase. FIG. 11B is a bar graph showing the statistical result of the normalized firing rate of THB6 compounds.

### Example 12. The effect of systemic injection of THB6 on mouse VTA DA neuron firing.

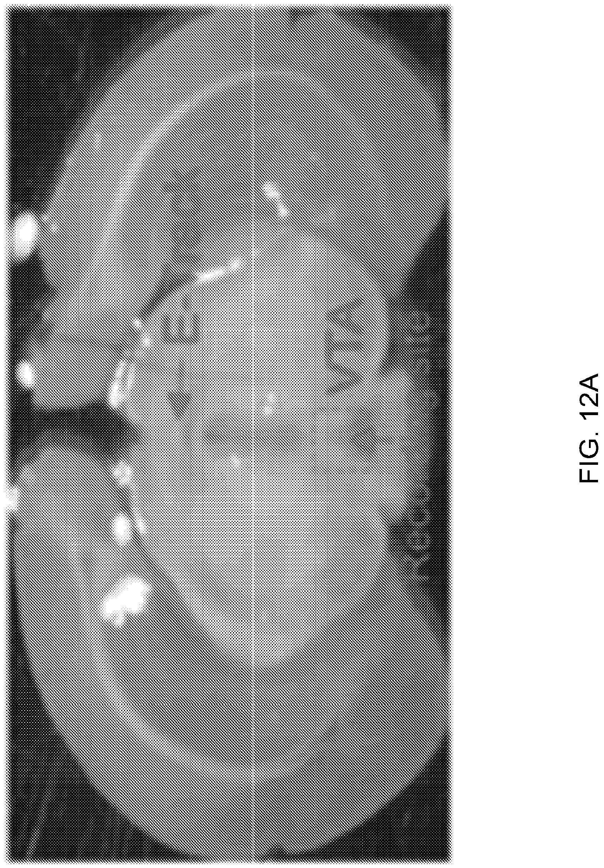

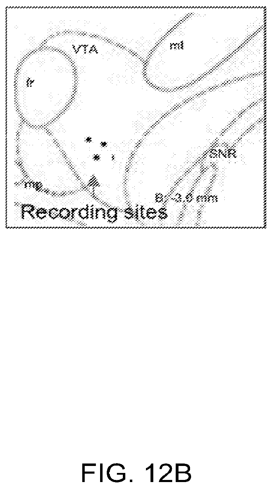

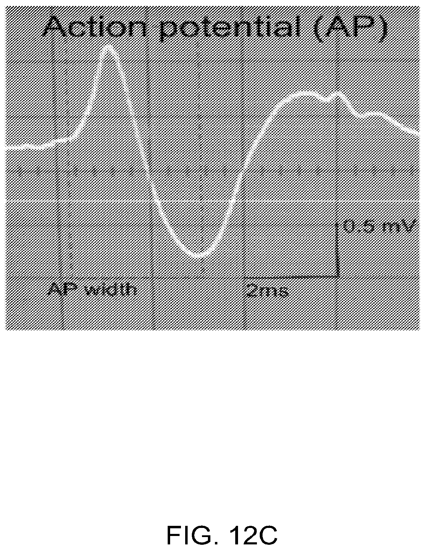

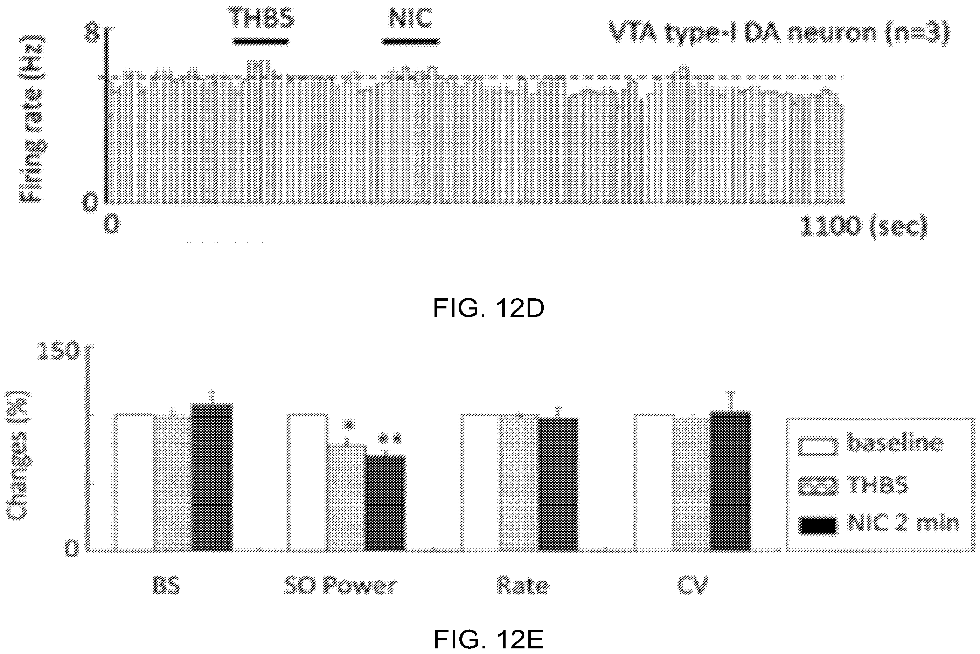

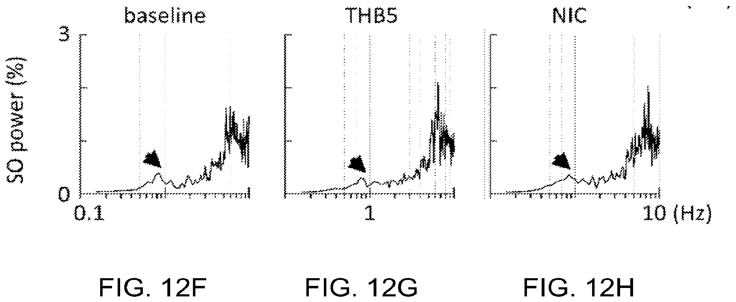

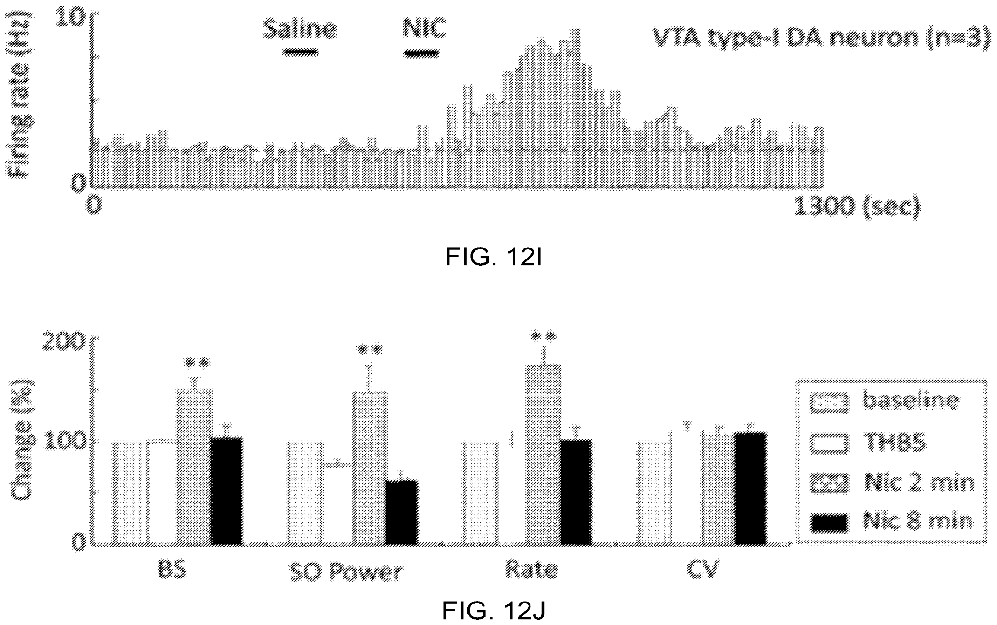

The effect of systemic injection of THB6 on mouse VTA DA neuron firing was evaluated by applying 10 mg/kg via an IV to anesthetized mice and extracellular single unit recordings were performed. FIG. 12A provides an image of the VTA slice, showing the recording electrode track and recording site after the experiment. The location of the recording electrode track is depicted in FIG. 12B. As seen in FIG. 12C, a single extracellular action potential (AP) with long duration time, suggesting a DA neuron. As seen in FIGS. 12D and 12E, THB6 alone did not affect neuronal firing rate, but prevented nicotine-induced increase of firing rate. Spectrum analysis, as shown in FIGS. 12F-12H, illustrates the effects of THB6 on DA firing slow oscillations. FIGS. 12I and 12J are graphs showing that systemic injection nicotine increases neuronal firing rate at 2 minutes after injection, but return back to normal level after 8 minutes.

### Example 13. The effect of systemic injection of THB6 on mouse VTA DA neuron firing.

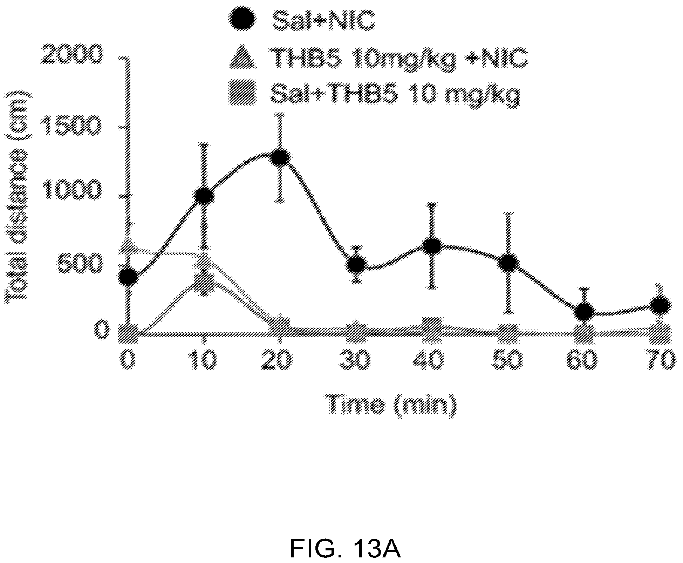

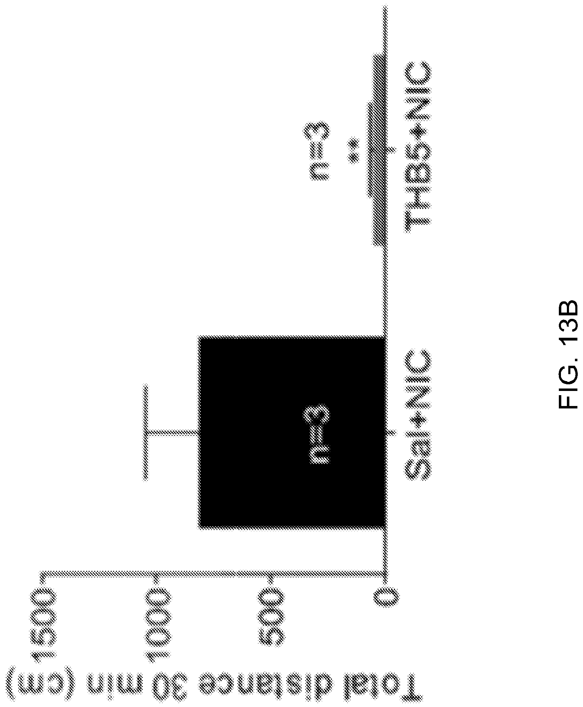

Systemic injection of dosages containing THB6 to mice and the mice's locomotive activity was studied. FIG. 13A is a graph showing that injections of dosages containing THB6 alone did not alter locomotor activity, but pretreatment of dosages containing THB6 with nicotine prevented nicotine-induced increase of locomotor activity. FIG. 13B shows the statistic result that THB6 prevents nicotine-induced increase of mouse locomotor activity.

### Example 14. Preparation of berberine compounds of Formula (I).

Five compounds were prepared according to the following synthesis steps. One of ordinary skill would understand how to make and utilize the following compounds based on the instant disclosure and the following synthesis steps.

As a preliminary step, Compound A was produced by mixing berberine hydrochloride (5 g, 0.013 mol) and phloroglucin (5 g, 0.04 mol) with 60% H₂SO₄ (40 mL) and stirred at 95° C in an oil bath for 20 min. The mixture was cooled, concentrated, and resuspended in H₂O/acetone (1:1), and then the intermediate was purified by silica gel chromatography (CHCl₃/MeOH, 15:1) to obtain 2.18 g. The reaction yield was 44%. A mixture of the intermediate (2.18 g, 0.006 mol) and MeOH (45 mL) was refluxed for 20 min to dissolve, then NaBH₄ (1.13 g, 0.03 mol) was added in small portions with stirring at room temperature. Two hours later, the resulting solids were filtered, washed with MeOH and purified by silica gel chromatography (CHCl₃/MeOH, 25:1) to obtain compound A 1.05 g (53%). Without being limited to any specific theory, Compound A was prepared in accordance with the following reactions:

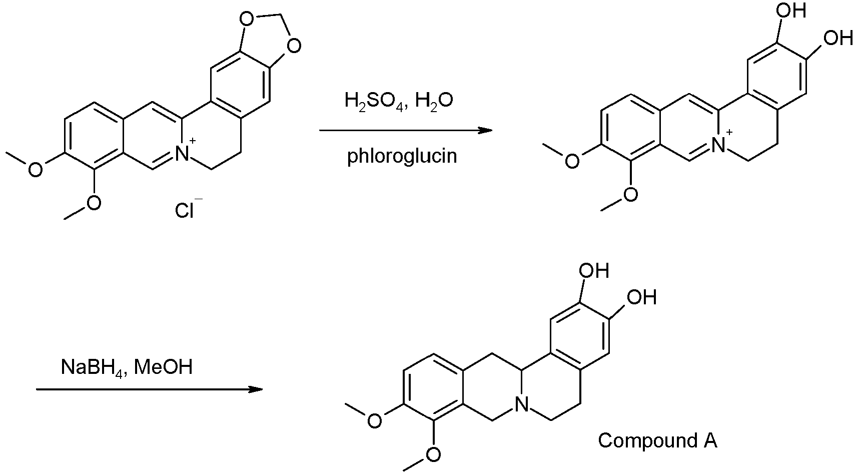

THB6, having a structure shown in Example 8, was produced by adding ethyldiisopropylamine (1.15 g, 9 mmol) and triphosgene (445 mg, 1.5 mmol) to a solution of compound A (327 mg, 1 mmol) in 10 mL of THF. This combined solution was stirred overnight at room temperature. Solvent was evaporated. 20 mL of DCM and 10 mL of distilled water was added to the residue. DCM solution was separated and washed twice with 10 mL of water. The organic solution was then dried over anhydrous Na₂SO₄, filtered, and evaporated to yield 95 mg of compound 5. The reaction yield was 27%.

Compounds of formula (I) were produced having a structure according to Formula (II):

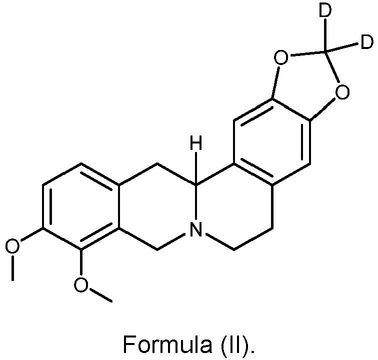

The compounds of Formula (II) were prepared by heating Compound A (327 mg, 1 mmol), Cs₂CO₃ (488 mg, 1.5 mmol ), CD₂Cl₂ (188 mg, 2 mmol) and 3 ml of DMF in sealed tube at 100 °C for 10 hours, purified by silica gel chromatography (CHCl3/MeOH, 25:1) to obtain 10.11 g. The reaction yield was 33%.

Compounds of Formula (I) were produced having a structure a structure according to Formula (III):

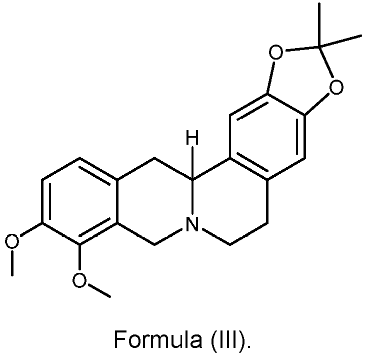

Specifically, Compound A (327 mg, 1 mmol) was converted to TFA-salt, which was dissolved in 5 mL of THF and 10 mL of acetone. 1.3 g of P₄O₁₀ was then added, and the mixture refluxed for 2 hours. 1 g of P₄O₁₀ was then added and the solution was refluxed for 2 hours. An additional 1 g of P₄O₁₀ was then added again and refluxed for 2 hour. The solution was cooled, neutralized with aqueous NaHCO₃, extracted with DCM, and then purified by silica gel chromatography (CHCl₃/MeOH, 25:1) to obtain compound 2 0.05 g (13%).

Compounds of Formula (I) were prepared having a structure according to Formula (IV):

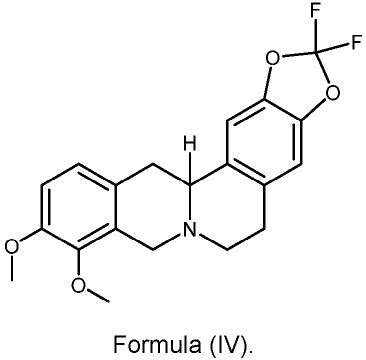

To produce the compounds of Formula (IV), Compound A (327 mg, 1 mmol), Cs₂CO₃ (488 mg, 1.5 mmol ), CF₂Br₂ (627 mg, 3 mmol) and 3 ml of DMF were heated in sealed tube at 95 °C for 16 hours, and then purified by silica gel chromatography (CHCl3/MeOH, 25:1) to obtain 10 mg. The reaction yield was 3%.

Compounds of Formula (I) were prepared having structure according to Formula (V):

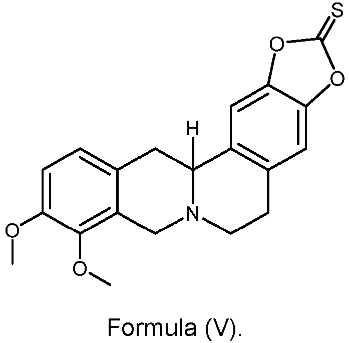

To produce the compounds of Formula (V), ethyldiisopropylamine (388 mg, 3 mmol) and thiocarbonyldiimidazole (534 mg, 3 mmol) to a solution of compound A (327 mg, 1 mmol) in 10 mL of THF. This solution was stirred for 40 hours at room temperature. Solvent was evaporated and 20 mL of DCM and 10 mL of distilled water was added to the residue. DCM solution was separated and washed twice with 10 mL of water. The organic solution was dried with anhydrous Na₂SO₄, filtered, and evaporated to yield 105 mg of compound 4. The reaction yield was 30%.

## Claims

1. A compound of Formula (I) or a salt thereof: 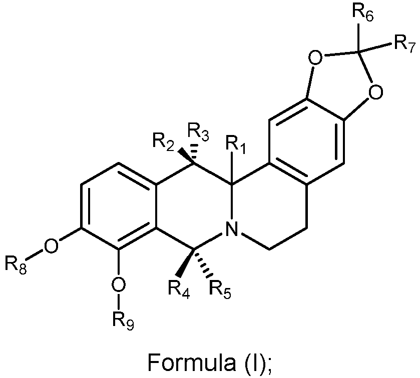
wherein R₁, R₂, R₃, R₄, and R₅ are independently H or D;
R₆ and R₇ are independently halogen, C₄ to C₈ unsubstituted aryl, C₂ to C₆ unsubstituted heterocycle, C₁ to C₆ unsubstituted alkyl, C₃-C₁₀ unsubstituted cycloalkyl, or R₆ and R₇ are taken together as =O or =S; and
R₈ and R₉ are independently selected from a group consisting of methyl, CHF₂, and CF₃.

2. The compound of claim 1, wherein R₁, R₂, R₃, R₄, and R₅ are independently hydrogen or deuterium; R₆ and R₇ are independently halogen, C₅ to C₇ unsubstituted aryl, C₄ to C₆ unsubstituted heterocycle, C₁ to C₄ unsubstituted alkyl, C₅-C₇ unsubstituted cycloalkyl, or R₆ and R₇ are taken together as =O or =S; and
R₈ and R₉ are independently selected from a group consisting of methyl, CHF₂, and CF₃.

3. The compound of claim 1, wherein R₁, R₂, R₃, R₄, and R₅ are independently hydrogen or deuterium; R₆ and R₇ are independently chlorine, fluorine, bromide, phenyl, methyl, ethyl, n-propyl, i-propyl, n-butyl, t-butyl, cyclopentyl, cyclohexyl, or R₆ and R₇ are taken together as =O or =S; and
R₈ and R₉ are methyl.

4. The compound of claim 1, wherein R₁, R₂, R₃, R₄, and R₅ are hydrogen; and R₈ and R₉ are methyl

5. The compound of claim 1, wherein R₁, R₂, R₃, R₄, and R₅ are hydrogen; R₆ and R₇ are methyl; and R₈ and R₉ are methyl, optionally wherein the compound of Formula (I) has a structure according to Formula (III):

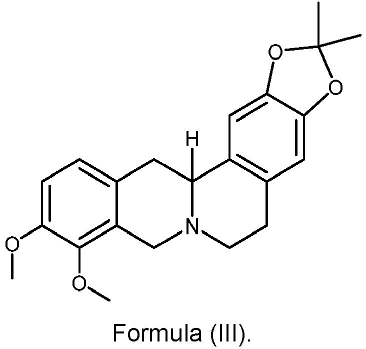

6. The compound of claim 1, wherein R₁, R₂, R₃, R₄, and R₅ are hydrogen; R₆ and R₇ are fluorine; and R₈ and R₉ are methyl, optionally wherein the compound of Formula (I) has a structure according to Formula (IV):

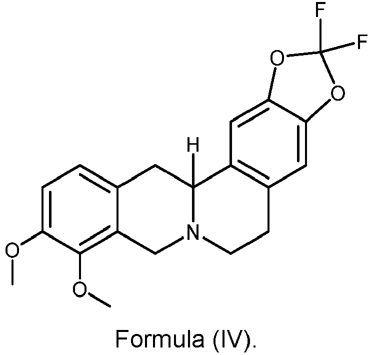

7. The compound of claim 1, wherein R₁, R₂, R₃, R₄, and R₅ are hydrogen; R₆ and R₇ are taken together as =S; and R₈ and R₉ are methyl, optionally wherein the compound of Formula (I) has a structure according to Formula (V):

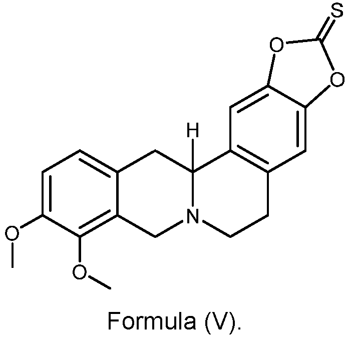

8. The compound of claim 1, wherein R₁, R₂, R₃, R₄, and R₅ are hydrogen; R₆ and R₇ are taken together as =O; and R₈ and R₉ are methyl, optionally wherein the compound of Formula (I) has a structure according to Formula (VI):

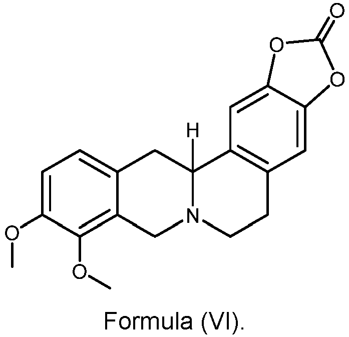

9. The compound of claim 1, wherein R₁, R₂, R₃, R₄, and R₅ are hydrogen; R₆ is methyl; R₇ is phenyl; and R₈ and R₉ are methyl, optionally wherein the compound of Formula (I) has a structure according to Formula (VII):

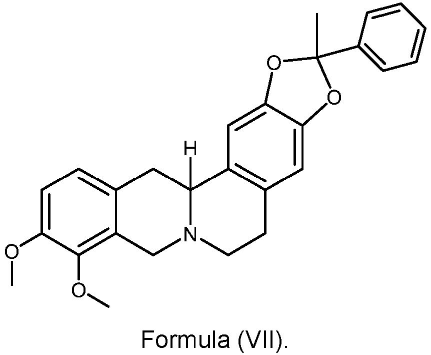

10. The compound of any one of the claims 1-9, wherein the compound of Formula (I) is a mixture of diastereomers, a single diastereomer, a racemic mixture, (R) enantiomer, or a (S) enantiomer.

11. The compound of any one of the claims 1-9, wherein the compound of Formula (I) is a (R) enantiomer, a (S) enantiomer, or a racemic mixture.

12. A berberine composition comprising an amount of the compound of claim 1.

13. The berberine composition of claim 12, wherein:
a) the composition comprises a therapeutically effective amount of the compound of Formula (I), optionally wherein the therapeutically effective amount of the compound comprising Formula (I) is more than about 1 µg; and/or
b) the composition comprises at least one excipient.

14. The berberine composition of claims 12 or 13, wherein the composition further comprises at least one tetrahydroprotoberberine, tetrahydropalmatine, stepholidine, or a combination of two or more thereof, optionally wherein the tetrahydropalmatine is *I*-tetrahydropalmatine, or wherein the stepholidine is *I*-stepholidine.

15. The composition of any one of claims 12-14 for use as a medicament.

16. The composition of any one of claims 12-14 for use in a method for reducing nicotine addiction in a subject.

17. The composition for use according to claim 16, wherein the composition reduces K_{ATP} channel signaling in an individual, optionally wherein the K_{ATP} channel signaling is part of a dopaminergic receptor, an adrenergic receptor, and/or a serotonin receptor, more optionally wherein the dopaminergic receptor is a D1, D2, D3, or D4 receptor subtype.

## Patentansprüche

1. Verbindung der Formel (I) oder ein Salz davon: 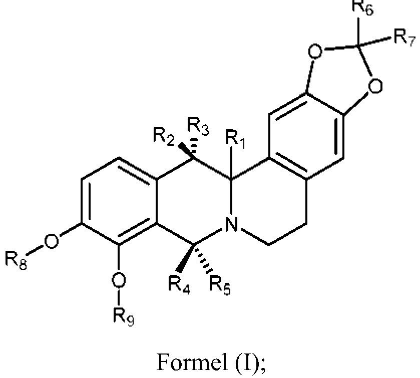
wobei R₁, R₂, R₃, R₄ und R₅ unabhängig H oder D sind;
R₆ und R₇ unabhängig Halogen, unsubstituiertes C₄- bis C₈-Aryl, unsubstituierter C₂- bis C₆-Heterocyclus, unsubstituiertes C₁- bis C₆-Alkyl, unsubstituiertes C₃-C₁₀-Cycloalkyl sind oder R₆ und R₇ als =O oder =S zusammengenommen sind; und
R₈ und R₉ unabhängig ausgewählt sind aus einer Gruppe bestehend aus Methyl, CHF₂ und CF₃.

2. Verbindung nach Anspruch 1, wobei R₁, R₂, R₃, R₄ und R₅ unabhängig Wasserstoff oder Deuterium sind; R₆ und R₇ unabhängig Halogen, unsubstituiertes C₅- bis C₇-Aryl, unsubstituierter C₄- bis C₆-Heterocyclus, unsubstituiertes C₁- bis C₄-Alkyl, unsubstituiertes C₅-C₇-Cycloalkyl sind oder R₆ und R₇ als =O oder =S zusammengenommen sind; und
R₈ und R₉ unabhängig ausgewählt sind aus einer Gruppe bestehend aus Methyl, CHF₂ und CF₃.

3. Verbindung nach Anspruch 1, wobei R₁, R₂, R₃, R₄ und R₅ unabhängig Wasserstoff oder Deuterium sind; R₆ und R₇ unabhängig Chlor, Fluor, Bromid, Phenyl, Methyl, Ethyl, n-Propyl, i-Propyl, n-Butyl, t-Butyl, Cyclopentyl, Cyclohexyl sind oder R₆ und R₇ als =O oder =S zusammengenommen sind; und
R₈ und R₉ Methyl sind.

4. Verbindung nach Anspruch 1, wobei R₁, R₂, R₃, R₄ und R₅ Wasserstoff sind; und R₈ und R₉ Methyl sind.

5. Verbindung nach Anspruch 1, wobei R₁, R₂, R₃, R₄ und R₅ Wasserstoff sind; R₆ und R₇ Methyl sind; und R₈ und R₉ Methyl sind, wobei optional die Verbindung der Formel (I) eine Struktur gemäß Formel (III) aufweist:

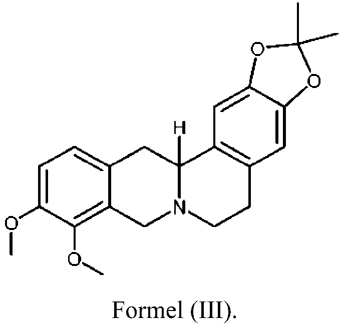

6. Verbindung nach Anspruch 1, wobei R₁, R₂, R₃, R₄ und R₅ Wasserstoff sind; R₆ und R₇ Fluor sind; und R₈ und R₉ Methyl sind, wobei optional die Verbindung der Formel (I) eine Struktur gemäß Formel (IV) aufweist:

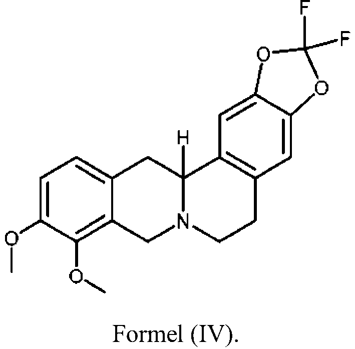

7. Verbindung nach Anspruch 1, wobei R₁, R₂, R₃, R₄ und R₅ Wasserstoff sind; R₆ und R₇ als =S zusammengenommen sind; und R₈ und R₉ Methyl sind, wobei optional die Verbindung der Formel (I) eine Struktur gemäß Formel (V) aufweist:

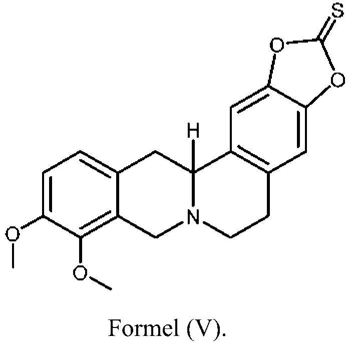

8. Verbindung nach Anspruch 1, wobei R₁, R₂, R₃, R₄ und R₅ Wasserstoff sind; R₆ und R₇ als =O zusammengenommen sind; und R₈ und R₉ Methyl sind, wobei optional die Verbindung der Formel (I) eine Struktur gemäß Formel (VI) aufweist:

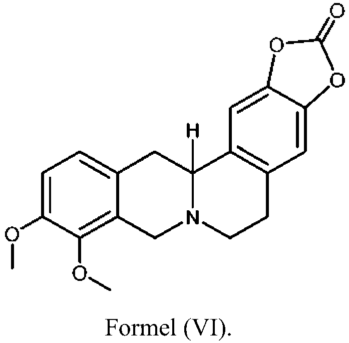

9. Verbindung nach Anspruch 1, wobei R₁, R₂, R₃, R₄ und R₅ Wasserstoff sind; R₆ Methyl ist; R₇ Phenyl ist; und R₈ und R₉ Methyl sind, wobei optional die Verbindung der Formel (I) eine Struktur gemäß Formel (VII) aufweist:

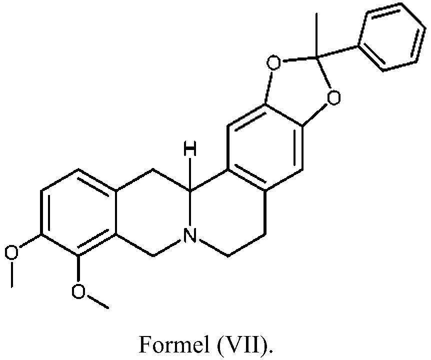

10. Verbindung nach einem der Ansprüche 1-9, wobei die Verbindung der Formel (I) ein Gemisch von Diastereomeren, ein einzelnes Diastereomer, ein racemisches Gemisch, ein (R)-Enantiomer oder ein (S)-Enantiomer ist.

11. Verbindung nach einem der Ansprüche 1-9, wobei die Verbindung der Formel (I) ein (R)-Enantiomer, ein (S)-Enantiomer oder ein racemisches Gemisch ist.

12. Berberinzusammensetzung, umfassend eine Menge der Verbindung nach Anspruch 1.

13. Berberinzusammensetzung nach Anspruch 12, wobei:
a) die Zusammensetzung eine therapeutisch wirksame Menge der Verbindung der Formel (I) umfasst, wobei optional die therapeutisch wirksame Menge der Verbindung, die Formel (I) umfasst, mehr als etwa 1 µg beträgt; und/oder
b) die Zusammensetzung mindestens einen Hilfsstoff umfasst.

14. Berberinzusammensetzung nach Anspruch 12 oder 13, wobei die Zusammensetzung ferner mindestens ein Tetrahydroprotoberberin, Tetrahydropalmatin, Stepholidin oder eine Kombination aus zwei oder mehr davon umfasst, wobei das Tetrahydropalmatin optional 1-Tetrahydropalmatin ist oder wobei das Stepholidin *l*-Stepholidin ist.

15. Zusammensetzung nach einem der Ansprüche 12-14 zur Verwendung als ein Medikament.

16. Zusammensetzung nach einem der Ansprüche 12-14 zur Verwendung in einem Verfahren zum Reduzieren von Nikotinabhängigkeit bei einem Subjekt.

17. Zusammensetzung zur Verwendung nach Anspruch 16, wobei die Zusammensetzung eine K_{ATP}-Kanalsignalisierung in einem Individuum reduziert, wobei optional die K_{ATP}-Kanalsignalisierung Teil eines dopaminergen Rezeptors, eines adrenergen Rezeptors und/oder eines Serotoninrezeptors ist, wobei stärker optional der dopaminerge Rezeptor ein D1-, D2-, D3- oder D4-Rezeptor-Subtyp ist.

## Revendications

1. Composé de formule (1), ou sel de celui-ci : 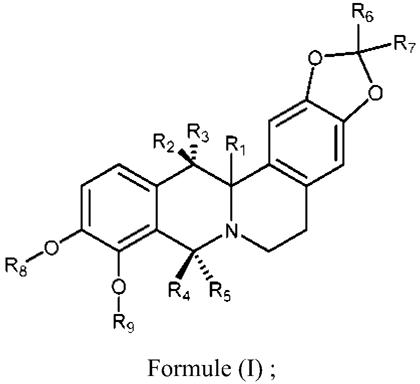
dans lequel R₁, R₂, R₃, R₄ et R₅ sont indépendamment H ou D ;
R₆ et R₇ sont indépendamment halogène, aryle en C₄ à C₈ non substitué, hétérocycle en C₂ à C₆ non substitué, alkyle en C₁ à C₆ non substitué, cycloalkyle en C₃-C₁₀, non substitué, ou R₆ et R₇ sont pris conjointement en tant que =O ou =S ; et
R₈ et R₉ sont indépendamment choisis dans un groupe constitué de méthyle, CHF₂ et CF₃.

2. Composé de la revendication 1, dans lequel R₁, R₂, R₃, R₄ et R₅ sont indépendamment hydrogène ou deutérium ; R₆ et R₇ sont indépendamment halogène, aryle en C₅ à C₇ non substitué, hétérocycle en C₄ à C₆ non substitué, alkyle en C₁ à C₄ non substitué, cycloalkyle en C₅-C₇ non substitué, ou R₆ et R₇ sont pris conjointement en tant que =O ou =S ; et
R₈ et R₉ sont indépendamment choisis dans un groupe constitué de méthyle, CHF₂ et CF₃.

3. Composé de la revendication 1, dans lequel R₁, R₂, R₃, R₄ and R₅ sont indépendamment hydrogène ou deutérium ; R₆ et R₇ sont indépendamment chlore, fluor, bromure, phényle, méthyle, éthyle, *n*-propyle, *i*-propyle, *n*-butyle, *t*-butyle, cyclopentyle, cyclohexyle, ou R₆ et R₇ sont pris conjointement en tant que =O ou =S ; et
R₈ et R₉ sont méthyle.

4. Composé de la revendication 1, dans lequel R₁, R₂, R₃, R₄ et R₅ sont hydrogène ; et R₈ et R₉ sont méthyle.

5. Composé de la revendication 1, dans lequel R₁, R₂, R₃, R₄ et R₅ sont hydrogène ; R₆ et R₇ sont méthyle ; et R₈ et R₉ sont méthyle, éventuellement dans lequel le composé de Formule (I) présente une structure selon la Formule (III) :

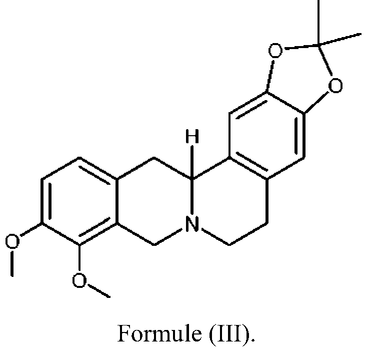

6. Composé de la revendication 1, dans lequel R₁, R₂, R₃, R₄ et R₅ sont hydrogène ; R₆ et R₇ sont fluor ; et R₈ et R₉ sont méthyle, éventuellement dans lequel le composé de Formule (I) présente une structure selon la Formule (IV) :

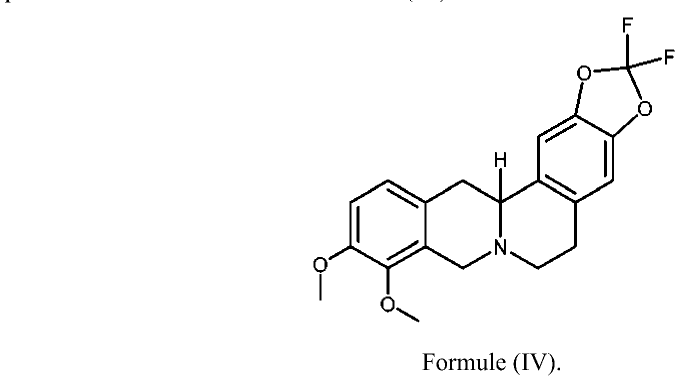

7. Composé de la revendication 1, dans lequel R₁, R₂, R₃, R₄ et R₅ sont hydrogène ; R₆ et R₇ sont pris conjointement en tant que =S ; et R₈ et R₉ sont méthyle, éventuellement dans lequel le composé de Formule (I) présente une structure selon la formule (V) :

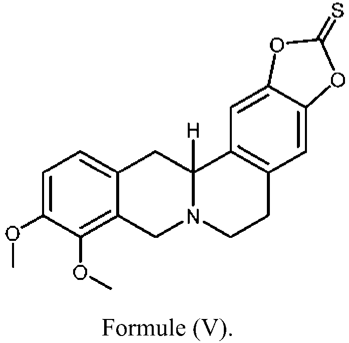

8. Composé de la revendication 1, dans lequel R₁, R₂, R₃, R₄ et R₅ sont hydrogène ; R₆ et R₇ sont pris conjointement en tant que =O ; et R₈ et R₉ sont méthyle, éventuellement dans lequel le composé de Formule (I) présente une structure selon la Formule (VI) :

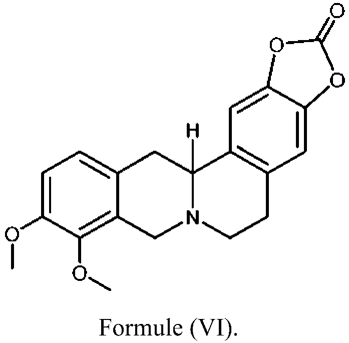

9. Composé de la revendication 1, dans lequel R₁, R₂, R₃, R₄ et R₅ sont hydrogène ; R₆ est méthyle ; R₇ est phényle ; et R₈ et R₉ sont méthyle, éventuellement dans lequel le composé de Formule (I) présente une structure selon la Formule (VII) :

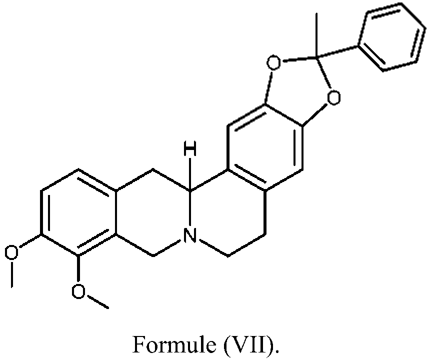

10. Composé de l'une quelconque des revendications 1 à 9, dans lequel le composé de Formule (I) est un mélange de diastéréomères, un diastéréomère unique, un mélange racémique, un énantiomère (R) ou un énantiomère (S).

11. Composé de l'une quelconque des revendications 1 à 9, dans lequel le composé de Formule (I) est un énantiomère (R), un énantiomère (S) ou un mélange racémique.

12. Composition de berbérine comprenant une quantité du composé de la revendication 1.

13. Composition de berbérine de la revendication 12, dans laquelle :
a) la composition comprend une quantité thérapeutiquement efficace du composé de Formule (I), éventuellement dans laquelle la quantité thérapeutiquement efficace du composé comprenant la Formule (I) est supérieure à environ 1 µg ; et/ou
b) la composition comprend au moins un excipient.

14. Composition de berbérine de la revendication 12 ou 13, dans laquelle la composition comprend en outre au moins une tétrahydroprotoberbérine, tétrahydropalmatine, stépholidine, ou une combinaison de deux ou plus de celles-ci, éventuellement ladite tétrahydropalmatine étant /tétrahydropalmatine, ou ladite stépholidine étant /-stépholidine.

15. Composition de l'une quelconque des revendications 12 à 14 destinée à être utilisée en tant que médicament.

16. Composition de l'une quelconque des revendications 12 à 14 destinée à être utilisée dans un procédé de réduction de la dépendance à la nicotine chez un sujet.

17. Composition destinée à être utilisée selon la revendication 16, dans laquelle la composition réduit la signalisation du canal K_{ATP} chez un individu, éventuellement dans laquelle la signalisation du canal K_{ATP} fait partie d'un récepteur dopaminergique, d'un récepteur adrénergique et/ou d'un récepteur de la sérotonine, plus éventuellement dans laquelle le récepteur dopaminergique est un sous-type de récepteur D1, D2, D3 ou D4.
